(19) Europäisches Patentamt / European Patent Office / Office européen des brevets

(11) **EP 3 045 917 A2**

(12) **EUROPEAN PATENT APPLICATION**
published in accordance with Art. 153(4) EPC


(43) Date of publication:
**20.07.2016 Bulletin 2016/29**

(21) Application number: **14789584.1**

(22) Date of filing: **12.09.2014**

(51) Int Cl.:
***G01N 33/574*** $^{(2006.01)}$ ***A61P 35/04*** $^{(2006.01)}$

(86) International application number:
**PCT/ES2014/070699**

(87) International publication number:
**WO 2015/036643 (19.03.2015 Gazette 2015/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.09.2013 ES 201331335**



(71) Applicant: **Sierra Jiménez, Angels**
**08208 Sabadell, Barcelona (ES)**

(72) Inventor: **Sierra Jiménez, Angels**
**08208 Sabadell, Barcelona (ES)**

(74) Representative: **ABG Patentes, S.L.**
**Avenida de Burgos, 16D**
**Edificio Euromor**
**28036 Madrid (ES)**


(54) **MARKER FOR PREDICTING METASTASIS OF BREAST CANCER**


(57) The invention relates to an *in vitro* method for predicting metastasis in a subject diagnosed with breast cancer and treated with a taxane, to an *in vitro* method for selecting breast cancer patients having a risk of developing metastasis after treatment with a taxane and to an *in vitro* method for designing personalized therapy for a subject suffering breast cancer and being treated with a taxane based on determining the level of GRP94 and/or FN14. The invention also relates to a composition comprising a taxane and a GRP94 inhibitor and/or an FN14 inhibitor and to the use thereof in the treatment of brain metastasis.


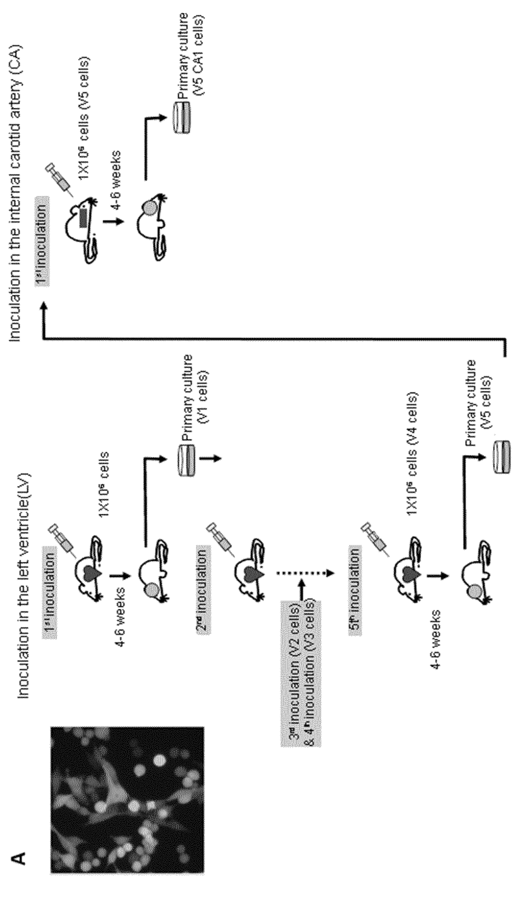


Figure 1




Printed by Jouve, 75001 PARIS (FR)

SEQUENCE LISTING

<110> ANGELS SIERRA JIMÉNEZ

<120> MARKER FOR PREDICTING BREAST CANCER METASTASIS

<130> P9443EPPC

<140> EP14789584.1
<141> 2014.09.12

<150> ES P201331335
<151> 2013-09-13

<160> 13

<170> PatentIn version 3.5

<210> 1
<211> 19
<212> DNA
<213>

<220>
<223> siRNA for GRP94

<400> 1
ugauguggau gguacagua 19

<210> 2
<211> 19
<212> DNA
<213> Artificial Sequence

<220>
<223> siRNA for GRP94

<400> 2
uacuguacca uccacauca 19

<210> 3
<211> 19
<212> DNA
<213> Artificial Sequence

<220>
<223> siRNA for GRP94

<400> 3
gaagaagcau cugauuacc 19

<210> 4
<211> 19
<212> DNA
<213> Artificial Sequence

<220>
<223> siRNA for GRP94

```
<400>  4
gccuuccaag ccgaaguua                                                    19


<210>  5
<211>  19
<212>  DNA
<213>  Artificial Sequence

<220>
<223>  siRNA for GRP94


<400>  5
ccuuccaagc cgaaguuaa                                                    19


<210>  6
<211>  19
<212>  DNA
<213>  Artificial Sequence

<220>
<223>  siRNA for GRP94


<400>  6
ucuggaaaug aggaacuaa                                                    19


<210>  7
<211>  19
<212>  DNA
<213>  Artificial Sequence

<220>
<223>  siRNA for GRP94


<400>  7
ccuugguacc auagccaaa                                                    19


<210>  8
<211>  19
<212>  DNA
<213>  Artificial Sequence

<220>
<223>  siRNA for GRP94

<400>  8
gcaucugauu accuvgaau                                                    19


<210>  9
<211>  19
<212>  DNA
<213>  Artificial Sequence

<220>
```

<223> siRNA for GRP94

<400> 9
ggagucugac uccaaugaa 19

<210> 10
<211> 21
<212> DNA
<213> Artificial Sequence

<220>
<223> siRNA of FN14

<400> 10
ggauucggcu ugguguugau u 21

<210> 11
<211> 21
<212> DNA
<213> Artificial Sequence

<220>
<223> siRNA of FN14

<400> 11
cgucguccau ucauucauuu u 21

<210> 12
<211> 21
<212> DNA
<213> Artificial Sequence

<220>
<223> siRNA of FN14

<400> 12
ggacugggcu uagaguucau u 21

<210> 13
<211> 21
<212> DNA
<213> Artificial Sequence

<220>
<223> siRNA of FN14

<400> 13
cuaaggaacu gcagcauuuu u 21

## Description

Object of the Invention

The present invention relates to *in vitro* methods for predicting metastasis in a subject diagnosed with breast cancer and treated with a taxane or for selecting said subject or for designing personalized therapy for said subject. The invention also relates to compositions and uses of the compositions for the treatment or prevention of brain metastasis.

Background of the Invention

Breast cancer is the second most common type of cancer (10.4%; after lung cancer) and the fifth most common cause of cancer death (after lung cancer, stomach cancer, liver cancer and colon cancer) worldwide. Among women, breast cancer is the most common cause of cancer death. In 2005, breast cancer caused 502,000 deaths worldwide (7% of cancer deaths; almost 1% of all deaths). The number of cases worldwide has increased significantly since the 1970s, a phenomenon partially attributed to modern lifestyles in the Western world.

Breast cancer is classified in stages according to the TNM system. The prognosis is closely linked to the results of stage classification, and stage classification is also used for assigning patients to treatments both in clinical trials and in medical practice. The information for stage classification is as follows:

- TX: The primary tumor cannot be evaluated. T0: No evidence of tumor. Tis: Carcinoma in situ, without invasion. T1: The tumor is 2 cm or smaller. T2: The tumor is bigger than 2 cm but smaller than 5 cm. T3: The tumor is bigger than 5 cm. T4: Tumor of any size growing in the skin or chest wall, or inflammatory breast cancer.
- NX: The neighboring lymph nodes cannot be evaluated. N0: The cancer has not spread to the regional lymph nodes. N1: The cancer has spread to 1 to 3 axillary lymph nodes or to an internal mammary node. N2: The cancer has spread to 4 to 9 axillary lymph nodes or to multiple internal mammary nodes. N3: One of the following applies: the cancer has spread to 10 or more axillary lymph nodes, or the cancer has spread to the lymph nodes below the clavicle, or the cancer has spread to the lymph nodes above the clavicle, or the cancer affects the axillary lymph nodes and has spread to the internal mammary lymph nodes, or the cancer affects 4 or more axillary lymph nodes, and minimum amounts of cancer are found in the internal mammary nodes or in sentinel lymph node biopsy.
- MX: The presence of distant spread (metastasis) cannot be evaluated. M0: No distant spread. M1: The cancer has spread to distant organs, not including the supraclavicular lymph node.

The fact that most patients with solid tumor cancer die due to subsequent metastasis means that it is crucial to understand the molecular and cellular mechanisms that allow the tumor to metastasize. Recent publications have demonstrated how metastasis occurs by means of complex mechanisms that are still relatively unknown and also how different metastatic cell types have a tropism towards specific organs. These specific tissue metastatic cells have a series of acquired functions that allow them to colonize specific organs.

Recent studies have demonstrated the expression of genes involved in brain metastasis of breast cancer, specifically HER2, EGFR, HPSE and Notch1 (Zhang L. et al., Science Translational Med, April 10, 2013, Vol. 5, Issue 180, p. 180ra48) and EGFR, p63 and Ki67 (Shao MM. Et al., Med Mol Morphol. 2011 Mar; 44(1):15-2).

There is a need to identify new markers which allow predicting the possibility of developing metastasis in subjects suffering breast cancer. The identification of new prognosis factors will serve as a guide for selecting the most suitable treatments.

Description of the Invention

In a first aspect, the invention relates to an *in vitro* method for predicting metastasis in a subject diagnosed with breast cancer and treated with a taxane comprising

i) determining the expression level of the GRP94 gene and/or the expression level of the FN14 gene in a sample from said subject, and
ii) comparing the expression level obtained in step i) with a reference value for each gene

wherein an increased expression level of GRP94 and/or an increased level of FN14 with respect to said reference value is indicative of a high risk of developing metastasis, or wherein a decreased expression level of GRP94 and/or a decreased level of FN14 with respect to said reference value is indicative of a low risk of developing metastasis.

In a second aspect, the invention relates to an *in vitro* method for selecting breast cancer patients having a risk of developing metastasis after treatment with a taxane comprising

i) determining the expression level of the GRP94 gene and/or the expression level of the FN14 gene in a sample from said subject, and
ii)comparing the expression level obtained in step i) with a reference value for each gene

wherein a decreased expression level of GRP94 and/or a decreased level of FN14 with respect to said reference value is indicative that said patient has a low risk of developing metastasis after treatment with a taxane.
In a third aspect, the invention relates to an *in vitro* method for designing personalized therapy for a subject suffering breast cancer and being treated with a taxane comprising

i) determining the expression level of the GRP94 gene and/or the expression level of the FN14 gene in a sample from said subject, and
ii) comparing the expression level obtained in step i) with a reference value for each gene

wherein if the expression level of GRP94 and/or the level of FN14 is decreased with respect to said reference value, then said subject is not susceptible to be treated with an agent suitable for the treatment of brain metastasis, or wherein if the expression level of GRP94 and/or the level of FN14 is increased with respect to said reference value, then said subject is susceptible to receive treatment with an agent suitable for the treatment of brain metastasis.
In a fourth aspect, the invention relates to the use of an agent suitable for the treatment of brain metastasis for manufacturing a medicinal product for the treatment or prevention of brain metastasis in a subject who has increased levels of GRP94 with respect to a reference value and has been treated with a taxane.
In a fifth aspect, the invention relates to the use of a taxane for manufacturing a medicinal product for the treatment of brain metastasis in a subject who has decreased levels of GRP94 with respect to a reference value.
In a sixth aspect, the invention relates to a composition comprising a taxane and a GRP94 inhibitor and/or an FN14 inhibitor, wherein if the taxane is docetaxel, then the FN14 inhibitor is not the compound of formula (I) of Table 2.
In a seventh aspect, the invention relates to the use of a composition of the invention for manufacturing a medicinal product for the treatment of brain metastasis.

Description of the Drawings

Figure 1 shows a scheme for obtaining highly brain metastatic cells by consecutive *in vivo*/*in vitro* implants whereby BRV5 cells are obtained after five passes, and then BRV5CA1 cells are obtained when they are inoculated in the internal carotid artery.
Figure 2 shows the cytotoxicity of taxotere on breast cancer cells which metastasize to the brain, BRV5CA1, and express the grp94 protein (pool) or have silenced expression (Clones 424-2 and 424-8). The response of the cells to treatment for 72 h in the presence of 1-10 ng/ml of taxotere is expressed as percentage of survival with respect to the control.
Figure 3 shows the relationship between the expression of GRP94 and the response to taxotere. Progression of the size of intramammary xenografts. The graph shows the medians of the variable response (normalized volume divided by the initial volume in day 0) over time for taxotere (TXT) and without treatment (CTR).
Figure 4(A) shows the graph corresponding to comparative ROC curves of the markers GRP94, FN14, GRP94+ FN14 and GRP94+FN14+HER2 in predicting the risk of brain metastasis. The graph corresponding to the ROC curves of the markers GRP94+FN14 (B) in predicting the risk of brain metastasis. The ROC curve graphs for FN14 (C) and GRP94(D).
Figure 5 shows the graphs depicting stratification of breast cancer patients overexpressing (top) or not overexpressing FN14 (bottom) treated or not treated with taxanes.
Figure 6 shows the expression of FN14 in triple-negative breast carcinoma grafts in immunosuppressed mice treated or not treated with lenalidomide (LND).
Figure 7 shows the effect of treatment with FN14 inhibitor on the growth of brain metastases.

Detailed Description of the Invention

The authors of the present invention have identified GRP94 and FN14 as genes related to the brain metastasis of breast cancer cells. Therefore, GRP94 and FN14 alone or in combination are useful for predicting the risk of brain metastasis. Furthermore, treatment of breast cancer cells metastasizing to the brain and having silenced expression of GRP94 with taxotere significantly reduces the survival of said cells.

Methods for the prognosis and selection of patients of the invention

In one aspect, the invention relates to an *in vitro* method for predicting metastasis in a subject diagnosed with breast cancer and treated with a taxane (first method of the invention) comprising

i) determining the expression level of the GRP94 gene and/or the expression level of the FN14 gene in a sample from said subject, and
ii)comparing the expression level obtained in step i) with a reference value for each gene

wherein an increased expression level of GRP94 and/or an increased level of FN14 with respect to said reference value is indicative of a high risk of developing metastasis, or wherein a decreased expression level of GRP94 and/or a decreased level of FN14 with respect to said reference value is indicative of a low risk of developing metastasis.

In another aspect, the invention relates to an *in vitro* method for selecting breast cancer patients having a risk of developing metastasis after treatment with a taxane (second method of the invention) comprising

i) determining the expression level of the GRP94 gene and/or the expression level of the FN14 gene in a sample from said subject, and
ii)comparing the expression level obtained in step i) with a reference value for each gene

wherein a decreased expression level of GRP94 and/or a decreased level of FN14 with respect to said reference value is indicative that said patient has a low risk of developing metastasis after treatment with a taxane.

In another aspect, the invention relates to an *in vitro* method for designing personalized therapy for a subject suffering breast cancer and being treated with a taxane (third method of the invention) comprising

i) determining the expression level of the GRP94 gene and/or the expression level of the FN14 gene in a sample from said subject, and
ii)comparing the expression level obtained in step i) with a reference value for each gene

wherein if the expression level of GRP94 and/or the level of FN14 is decreased with respect to said reference value, then said subject cannot be treated with an agent suitable for the treatment of brain metastasis, or wherein if the expression level of GRP94 and/or the level of FN14 is increased with respect to said reference value, then said subject can receive treatment with an agent suitable for the treatment of brain metastasis.

In the context of the present invention, "metastasis" is understood as the propagation of a localized cancer to an organ other than the organ where it started. It generally occurs through the blood or lymphatic system. When the cancerous cells spread and form a new tumor, the latter is called a secondary or metastatic tumor. The cancer cells forming the secondary tumor are like those of the original tumor. For example, if a breast cancer spreads (metastasizes) to the brain, the secondary tumor is made up of malignant breast cancer cells. In a particular embodiment of the first and second method of the invention, the metastasis is brain metastasis, i.e., breast cancer which has spread (metastasized) to the brain.

As it is used herein, "predicting" refers to the determination of the probability that the subject suffering breast cancer will develop metastasis in distant organs.

A person skilled in the art will understand that the prediction of the tendency of a primary breast tumor to metastasize does not need to be correct for all the subjects to be identified (i.e., for 100% of the subjects). Nevertheless, the term requires allowing the identification of a statistically significant part of the subjects (for example, a cohort in a cohort study). Whether a part is statistically significant can be determined in a simple manner by the person skilled in the art using various well-known statistical evaluation tools, for example, the determination of confidence intervals, determination of p-values, Student's T test, Mann-Whitney test, etc. Details are provided in Dowdy and Wearden, Statistics for Research, John Wiley and Sons, New York 1983. The preferred confidence intervals are at least 90%, at least 95%, at least 97%, at least 98% or at least 99%. The p-values are preferably 0.1, 0.05, 0.01, 0.005 and 0.0001. More preferably, at least 60%, at least 70%, at least 80% or at least 90% of the subjects of a population can be suitably identified by the method of the present invention.

As used herein, the term "subject" or "patient" refers to all animals classified as mammals and includes but is not limited to domestic and farm animals, primates and humans, for example, human beings, non-human primates, cows, horses, pigs, sheep, goats, dogs, cats or rodents. The subject is preferably a man or woman of any age or race, and more preferably the subject is a woman.

According to the methods of the invention, the subject suffers breast cancer. The person skilled in the art knows the different assays to find out whether or not a subject suffers breast cancer. For example, it is possible to use diagnostic imaging techniques, among others, mammography, breast ultrasound, magnetic resonance or magnetic resonance

imaging (MRI) or resonance, positron emission tomography (PET). Alternatively, the diagnosis can be obtained by means of biopsy.

As it is used herein, "breast cancer" refers to the uncontrolled growth of malignant cells in breast tissues. The types of breast cancer are mainly classified in 5 groups, i.e., ductal carcinoma *in situ,* infiltrating ductal carcinoma, lobular carcinoma *in situ,* infiltrating lobular carcinoma and inflammatory carcinoma. Ductal carcinoma *in situ* includes the galactophorous or mammary ducts. If left untreated, it can spread beyond the mammary ducts and cause metastasis. Invasive or infiltrating ductal carcinoma invades the breast adipose tissue from one of the ducts. Invasive carcinoma is the most common breast cancer; it makes up about 80% of all breast cancer cases. Lobular carcinoma *in situ* originates in the mammary glands (or lobes), and although it is not a real cancer, it increases the risk that the woman may develop a tumor in the future. Once it is detected, it is important for the woman to undergo a control mammography every year and several clinical examinations to monitor the possible development of cancer. Invasive lobular carcinoma follows the same filtration process as invasive ductal carcinoma towards the adipose tissue, but from lobules. Inflammatory carcinoma is less common but quite aggressive. It is referred to as inflammatory because the cancerous cells block the lymphatic vessels and this is shown on the skin, which acquires a thick and hollow appearance similar to that of an orange peel. Breast cancer is also understood as mucinous or colloid breast cancer or Paget's disease of the breast.

As it is used herein, "taxane" is understood as an antineoplastic medicinal product preventing cell growth by preventing cell division. It is also referred to as antimicrotubule, antimitotic and mitotic inhibitor. Taxanes are terpenes produced by plants of the genus Taxus, such as the Pacific Yew tree, hence their generic name. They were identified for the first time in natural sources, but some of them have been artificially synthesized. Taxanes suitable for use in the present invention include, without limitation, paclitaxel (Taxol®), docetaxel (Taxotere®), larotaxel, cabazitaxel, nab-paclitaxel (Abraxane®), paclitaxel dextrin, paclitaxel xylose, synthetic or semi-synthetic taxane derivatives, taxane derivatives such as IDN 5390, GRN1005, taxane derivatives described in EP 2330100A1, and taxane derivatives described or mentioned in Bioscience, Biotechnology, and Biochemistry, Vol. 76 (2012), No. 2 pp. 349-352. Paclitaxel and docetaxel are the preferred taxanes.

As it is used herein, "paclitaxel" or Taxol® refers to the compound 11-methano-5H-cyclodeca[3,4]benz[1,2-b]oxete benzenepropanoic acid deriv., with CAS number 33069-62-4.

As it is used herein, "docetaxel", also referred to as Taxotere®, refers to N-debenzoyl-N-tert-butoxycarbonyl-10-deacetyl taxol with CAS number 114977-28-5.

The methods of the invention comprise in a first step determining the expression level/levels of the GRP94 gene and/or FN14 gene in a sample from a subject.

As it is used herein, "GRP94", also referred to as endoplasmin, HSP90B1, gp96 or ERP99, refers to a gene encoding an endoplasmic reticulum chaperone with ATPase activity collaborating in the function of processing and transporting secreted proteins. The proteins have three important domains: one for binding to ATP, a protein domain and a dimerization domain. In humans, the GFRP94 gene encodes the protein corresponding to the P14625 sequence of the Uniprot database as of April 17, 2013.

As it is used herein, "FN14", also referred to as TNFRSF12A, CD266 or TWEAKR, refers to a gene encoding the fibroblast growth factor-inducible 14 kDa protein, tumor necrosis factor receptor superfamily member 12 A. FN14 encodes the TWEAK receptor and together they play a role in cell death and inflammation. In humans, the FN14 gene encodes the protein corresponding to the Q9NP84 sequence of the Uniprot database as of June 6, 2013.

As it is used herein, "sample" is understood as biological material isolated from a subject. The biological sample can contain any biological material suitable for determining the expression level of the GRP94 gene and/or FN14 gene. The sample can be isolated from any suitable biological tissue or fluid, such as, for example, tumor tissue, blood, blood plasma, serum, urine or cerebrospinal fluid. In a particular embodiment of the methods of the invention, the sample is a tumor tissue sample.

In the present invention, "tumor tissue sample" is understood as the tissue sample originating from the primary breast cancer tumor. The tumor tissue sample can be obtained by means of conventional methods, for example, biopsy, using methods well known by the persons skilled in related medical techniques. The methods for obtaining a biopsy sample include splitting a tumor into large pieces, or microdissection, or other cell separating methods known in the art. Tumor cells can additionally be obtained by means of cytology through fine needle-aspiration. To simplify sample preservation and handling, samples can be fixed in formalin and embedded in paraffin or be frozen first and then embedded in a medium that can be cryosolidified, such as OCT compound, by means of immersion in a highly cryogenic medium which allows rapid freezing.

As it is used herein, the term "expression level" of a gene refers to the measurable amount of gene product in a sample from the subject, in which the gene product can be a transcription product or a translation product. Accordingly, the expression level can correspond to a nucleic acid gene product, such as mRNA or cDNA, or a polypeptide gene product. The expression level is derived from the sample from a subject and/or reference sample or samples, and can be detected *de novo,* for example, or correspond to a previous determination.

As understood by the person skilled in the art, the expression level of the GRP94 gene and/or FN14 gene can

be quantified by means of quantifying the expression levels of the proteins encoded by said genes, i.e., in the case of GRP94 the endoplasmin protein or any functionally equivalent variant of the endoplasmin protein or by means of determining the activity of said protein, and in the case of FN14, the fn14 protein or any functionally equivalent variant of the fn14 protein or by means of determining the activity of said protein.

In the context of the present invention, "functionally equivalent variant of a protein" is understood as (i) variants of the protein in which one or more of the amino acid residues are substituted with a conserved or non-conserved amino acid residue (preferably a conserved amino acid residue), wherein such substituted amino acid residue may or may not be one encoded by the genetic code, or (ii) variants comprising an insertion or a deletion of one or more amino acids and having the same function as the protein, i.e., in the case of grp94, to act as a chaperone aiding in the folding of newly formed proteins, and in the case of fn14, to act as a TWEAK receptor.

Variants of the grp94 protein can be identified using methods based on the ATPase activity of grp4, such as that described in Dollins DE et al., Mol Cell. 2007 October 12; 28(1): 41-56, or methods based on the analysis of the chaperone function, such as those described in Ramsey, A. J., Russell, L. C., Whitt, S. R., and Chinkers, M. (2000) J. Biol. Chem. 275, 17857-17862 and Young, J. C., Schneider, C., and Hartl, F. U. (1997) FEBS Lett. 418, 139-143.

Variants of the fn14 protein can be identified, for example, using methods based on TWEAK binding capacity, or methods based on NF-kappaB activation capacity as described in Brown SA. et al., Biochem J. 2003 Apr 15; 371(Pt 2):395-403.

The variants of grp94 or the variants of fn14 according to the invention preferably have a sequence identity with the amino acid sequence of grp94 or with the sequence of fn14, respectively, of at least 50%, at least 60%, at least 70%, at least 80%, at least 90%, at least 91%, at least 92%, at least 93%, at least 94%, at least 95%, at least 96%, at least 97%, at least 98% or at least 99%. The degree of identity between the variants and the specific sequences of fn14 or grp94 protein defined previously is determined using algorithms and computing methods which are widely known for persons skilled in the art. The identity between two amino acid sequences is preferably determined using the BLASTP algorithm [BLAST Manual, Altschul, S., *et al.,* NCBI NLM NIH Bethesda, Md. 20894, Altschul, S., et al., J. Mol. Biol. 215: 403-410 (1990)].

The expression level of the grp94 protein and/or of the fn14 protein can be quantified by means of any conventional method which allows detecting and quantifying said protein in a sample from a subject. By way of non-limiting illustration the levels of grp94 or fn14 can be quantified, for example, by means of using antibodies capable of binding to grp94 (or to fragments thereof containing an antigenic determinant) or fn14, respectively, and the subsequent quantification of the complexes formed. The antibodies used in these assays may or may not be labeled. Illustrative examples of markers which can be used include radioactive isotopes, enzymes, fluorophores, chemiluminescence reagents, enzyme substrates or cofactors, enzyme inhibitors, particles, dyes, etc. There is a wide range of known assays which can be used in the present invention using unlabeled antibodies (primary antibody) and labeled antibodies (secondary antibody); these techniques include Western-blot transfer, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of protein microarrays or biochips including specific antibodies or assays based on colloidal precipitation in formats such as dipsticks. Other ways for detecting and quantifying the proteins include affinity chromatography techniques, ligand binding assays, etc. When an immunological method is used, any antibody or reagent that is known to bind to the grp94 protein with a high affinity can be used for detecting the amount thereof. However, the use of an antibody is preferred, for example, polyclonal sera, supernatants of hybridomas or monoclonal antibodies, antibody fragments, Fv, Fab, Fab' and F(ab')2, scFv, humanized diabodies, triabodies, tetrabodies and antibodies. There are commercial anti-grp94 protein antibodies on the market which can be used in the context of the present invention. There are many commercial companies offering anti-GRP94 antibodies, such as Novus Biologicals, Thermo Scientific, Pierce Antibodies, GeneTex and Abcam, among others.

In a particular embodiment, the levels of grp94 and/or fn14 protein are quantified by means of Western blot, ELISA or a protein array.

On the other hand, the expression level of the GRP94 gene and/or FN14 gene can also be quantified by means of quantifying the expression levels of a gene which can be determined by measuring the levels of messenger RNA of said gene or of the protein encoded by said gene.

For this purpose, the biological sample can be treated to physically or mechanically break up the cell or tissue structure, releasing the intracellular components into an aqueous or organic solution for preparing nucleic acids. The nucleic acids are extracted by means of commercially available methods known by the person skilled in the art (Sambrook, J., et al., "Molecular cloning: a Laboratory Manual", 3rd ed., Cold Spring Harbor Laboratory Press, N.Y., Vol. 1-3.).

Therefore, the expression level of the GRP94 gene and/or FN14 gene can be quantified based on the RNA resulting from the transcription of said gene (messenger RNA or mRNA), or alternatively, based on the complementary DNA (cDNA) of said gene. Therefore, in a particular embodiment of the methods of the invention, the quantification of the expression levels of the GRP94 gene comprises quantifying the messenger RNA of the GRP94 gene, or a fragment of said mRNA, complementary DNA of the GRP94 gene, or a fragment of said cDNA, or the mixtures thereof.

Virtually any conventional method can be used within the framework of the invention for detecting and quantifying the levels of mRNAs encoded by the FN14 and/or GRP94 gene or of the corresponding cDNA thereof. By way of non-limiting illustration, the levels of mRNA encoded by said gene can be quantified by means of using conventional methods, for example, methods comprising mRNA amplification and the quantification of said mRNA amplification product, such as electrophoresis and staining, or alternatively, by means of Southern blot and using suitable probes, Northern blot and using specific probes of the mRNA of the gene of interest (GRP94 and/or FN14) or of the corresponding cDNA thereof, mapping with S1 nuclease, RT-PCR, hybridization, microarrays, etc., preferably by means of real time quantitative PCR using a suitable marker. Similarly, the levels of cDNA corresponding to said mRNA encoded by the FN14 and/or GRP94 gene can also be quantified by means of using conventional techniques; in this case, the method of the invention includes a step of synthesizing the corresponding cDNA by means of reverse transcription (RT) of the corresponding mRNA followed by the amplification and quantification of said cDNA amplification product. Conventional methods for quantifying expression levels can be found, for example, in Sambrook et *al.,* 2001. (mentioned ad supra).

These methods are known and a person skilled in the art would be familiar with the normalizations necessary for each technique. For example, the expression measurements generated using multiplex PCR must be normalized by means of comparing the expression of the gene which is measured with the so-called "housekeeping" genes, the expression of which must be constant in all samples, thus providing a baseline expression to compare against, or with other control genes the expression of which are known to be modulated with cancer.

In a particular embodiment, the expression levels of the FN14 and/or GRP94 gene is quantified by means of a quantitative polymerase chain reaction (PCR) or a DNA or RNA array or by means of nucleotide hybridization techniques.

The second step of the methods of the invention comprises comparing the expression level obtained in step i) with a reference value.

As it is used herein, "reference value" refers to a value obtained in the laboratory and used as a reference for values or data obtained by means of laboratory examinations of patients or samples collected from patients. The reference value or reference level can be an absolute value, a relative value, a value having an upper and/or lower limit; a range of values, a mean value, a median value, an average value, or a value as compared to a specific control or reference value. A reference value can be based on an individual sample value, such as, for example, a value obtained from a sample from the subject being tested, but at an earlier point in time. The reference value can be based on a large number of samples, such as values from a population of subjects from the same age group, or based on a pool of samples including or excluding the sample to be tested.

In one embodiment, the reference value or values as understood herein can represent absolute amounts. In another embodiment, the expression level can be directly determined in relation to the reference value (for example, in terms of an increase or decrease, or an increase or decrease in the number of times).

In a preferred embodiment, the reference value of GRP94 is the expression level of the GRP94 gene in a control sample or reference sample, and the reference value of FN14 is the expression level of the FN14 gene in a control sample or reference sample. The exact nature of the control or reference sample can vary depending on the type of tumor to be analyzed.

Therefore, if a prognosis is to be evaluated, then the reference sample is a sample from a subject with breast cancer that has not metastasized or that corresponds with the median value of the gene expression level of GRP94 or FN14 measured in a tumor tissue collection of biopsy samples from patients with breast cancer that has not metastasized.

Said reference sample is typically obtained by combining equal amounts of samples from a population of subjects. Generally, the typical reference samples will be obtained from subjects who are clinically well-documented and in whom the absence of metastasis is well-characterized. In such samples, normal (reference) concentrations of the biomarker (FN14 and/or GRP94 gene) can be determined, for example, by providing the mean concentration over the reference population. Various considerations are taken into account when determining the reference concentration of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group. The sample collection from which the reference level is derived will preferably comprise subjects suffering from the same type of cancer as the patient object of study.

In a particular embodiment, the reference values for an "increased" or "decreased" expression of gene expression are determined by calculating the percentiles by conventional means which involves performing assays in one or several samples isolated from subjects whose disease is well documented by any of the methods mentioned above, the expression levels of GRP94 and/or FN14. The "decreased" levels of GRP94 and/or FN14 can then preferably be assigned to samples in which the expression levels of GRP94 and/or FN14 are equal to or less than the $50^{th}$ percentile in the normal population including, for example, expression levels equal to or less than the $60^{th}$ percentile in the normal population, equal to or less than the $70^{th}$ percentile in the normal population, equal to or less than the $80^{th}$ percentile in the normal population, equal to or less than the $90^{th}$ percentile in the normal population, and equal to or less than the $95^{th}$ percentile in the normal population. The "increased" expression levels of FN14and/or GRP94 gene can then preferably be assigned to samples in which the expression levels of the FN14 and/or GRP94 gene are equal to or greater than the $50^{th}$ percentile in the normal population including, for example, expression levels equal to or more than the $60^{th}$ percentile in the normal population, equal to or more than the $70^{th}$ percentile in the normal population, equal to or more than the $80^{th}$ percentile in the normal population, equal to or more than the $90^{th}$ percentile in the normal population, and equal to or more than the $95^{th}$ percentile in the normal population.

After performing steps i) and ii) of the first method of the invention, if the expression level of said gene is increase with respect to said reference value, it is indicative of a high risk of developing metastasis, or wherein if the expression level of said gene is decreased with respect to said reference value, it is indicative of a low risk of developing metastasis.

In the present invention, "increased expression levels" is understood as when the expression level of a gene (GRP94 or FN14) is greater than the reference value. Particularly, a sample can be considered to have high expression levels of GRP94 or FN14 when the expression levels in the sample from the subject are at least 1.1-fold, 1.5-fold, 5-fold, 10-fold, 20-fold, 30-fold, 40-fold, 50-fold, 60-fold, 70-fold, 80-fold, 90-fold, 100-fold or even more with respect to the reference value of GRP94 or FN14, respectively.

In the present invention, "decreased expression levels" is understood as when the expression level of a gene (GRP94 or FN14) is less than the reference value. Particularly, a sample can be considered to have low expression levels of GRP94 or FN14 when the expression levels in the sample from the subject have decreased by at least 1.1-fold, 1.5-fold, 5-fold, 10-fold, 20-fold, 30-fold, 40-fold, 50-fold, 60-fold, 70-fold, 80-fold, 90-fold or 100-fold with respect to the reference value of GRP94 or FN14, respectively.

"High risk" is understood as the situation in which the subject has at least a 20%, at least a 30%, at least a 40%, at least a 50%, at least a 60%, at least a 70%, at least an 80%, at least a 90%, at least a 100% probability of developing metastasis over time.

"Low risk" is understood as the situation in which the subject has less than a 20%, less than a 15%, less than a 10%, less than a 5% probability of developing metastasis over time.

After performing steps i) and ii) of the second method of the invention, if the expression level of GRP94 and/or FN14 is decreased with respect to said reference value, it is indicative that said patient has a low risk of developing metastasis after treatment with a taxane.

After performing steps i) and ii) of the third method of the invention, if the expression level of GRP94 and/or FN14 is decreased with respect to said reference value, then said subject is not treated with an agent suitable for the treatment of brain metastasis, or if the expression level of said gene is increased with respect to said reference value, then said subject can receive treatment with an agent suitable for the treatment of brain metastasis.

According to the present invention, "agents suitable for the treatment of brain metastasis" is understood as a compound capable of reducing or stopping the development of brain metastasis. Agents suitable for the treatment of brain metastasis for use according to the present invention are, among others, an anti-angiogenic agent, an hsp90 inhibitor, an epidermal growth factor inhibitor, an HDAC inhibitor, a PARP inhibitor, a BRAF inhibitor or a tyrosine kinase (TK) inhibitor. As the person skilled in the art will understand, the agents suitable for the treatment of brain metastasis according to the present invention include not only the compounds mentioned below but also derivatives of said compounds, as well as molecules acting by interrupting the VEGF, HSP90, EGF, HDAC, PARP, BRAF and TK signaling pathway.

As it is used herein, "anti-angiogenic agent" is understood as a chemical or biological agent inhibiting or reducing the formation of new blood vessels from pre-existing vessels (angiogenesis).

To find out if a compound is an anti-angiogenic agent, it is possible to use any assay known in the state of the art for detecting angiogenesis, such as those described in Auerbachj R. et al., Clin Chem. 2003 Jan; 49(1):32-40 or commercial assays such as Angiogenesis Assay of Cell Biolabs, Inc., such that if the compound to be assayed inhibits angiogenesis, said compound is an anti-angiogenic agent useful in the present invention.

In a particular embodiment, the anti-angiogenic agent is selected from the group consisting of bevacizumab, sunitinib, endostatin, sorafenib and cilengitide.

As it is used herein, "bevacizumab" refers to a humanized monoclonal antibody that binds to the vascular endothelial growth factor (VEGF), inhibiting VEGF from binding to its Flt1 and KDR receptors located on the surfaces of endothelial cells.

As it is used herein, "sunitinib" refers to the compound N-[2-(diethylamino)ethyl]-5-[(*Z*)-(5-fluoro-1,2-dihydro-2-oxo-3*H*-indol-3-ylidine)methyl]-2,4-dimethyl-1*H*-pyrrole-3-carboxamide with CAS number 341031-54-7. Sunitinib inhibits cell signaling by binding to several tyrosine kinase receptors (TKRs).

As it is used herein, "endostatin" or COL18A1 refers to a protein derived from a form of collagen corresponding to the protein with Uniprot database accession number P39060 as of April 17, 2013.

As it is used herein, "sorafenib" refers to the compound 4-[4-({[4-chloro-3-(trifluoromethyl)phenyl]carbamoyl}amino)phenoxy]-N-methylpyridine-2-carboxamide with CAS number 284461-73-0.

As it is used herein, "cilengitide" refers to the compound 2-[(2*S*,5*R*,8*S*,11*S*)-5-benzyl-11-{3-[(diaminomethylidene)amino]propyl}-7-methyl-3,6,9,12,15-pentaoxo-8-(propan-2-yl)-1,4,7,10,13-pentaazacyclopentadecan-2-yl]acetic

acid with CAS number 188968-51-6.

As it is used herein, "HSP90 inhibitor" refers to a compound capable of reducing the levels of protein or the activity of HSP90, in humans, the HSP90B1 isoform corresponds to the protein with Uniprot database accession number P14625 as of April 17, 2013. HSP90 is a chaperone protein which allows the correct folding of other proteins and stabilizes them against thermal stress and aids in protein degradation. There are several HSP90 isoforms, including, by way of non-limiting illustrative example, the HSP90B, TRAP and HSP90A family.

To find out if a compound is an HSP90 inhibitor, it is possible to use any assay known in the state of the art for analyzing HSP90 activity, such as the assays described in Aherne W. et al., Methods in Molecular Medicine | Volume: 85, 26 Mar, 2003, pages 149-161, or commercial kits such as Hsp90α Assay Kit of BPS Bioscience.

HSP90 inhibitors useful in the present invention are the compounds described in Table 1 below. In a particular embodiment, the inhibitor is 17-(dimethylaminoethylamino)-17-demethoxygeldanamycin.

As it is used herein, "epidermal growth factor inhibitor" refers to a compound capable of inhibiting the activity of epidermal growth factor (EGF), i.e., cell growth, proliferation and differentiation, by binding to the EGFR receptor.

To find out if a compound is an epidermal growth factor inhibitor, it is possible to use any assay known in the state of the art for analyzing cell proliferation in the presence of EGF, such as the commercial kit, Cell Proliferation Assay Kits of Life technologies, such that if the compound to be assayed inhibits cell proliferation in the presence of EGF, said compound is an EGF inhibitor.

As it is used herein, "trastuzumab" refers to a humanized monoclonal antibody targeting epidermal growth factor receptor 2 (HER2) with CAS number 180288-69-1.

As it is used herein, "erlotinib" refers to the compound N-(3-ethynylphenyl)-6,7-bis(2-methoxyethoxy)quinazolin-4-amine with CAS number 183321-74-6.

As it is used herein, "lapatinib" refers to the compound N-[3-chloro-4-[(3-fluorophenyl)methoxy]phenyl]-6-[5-[(2-methylsulfonylethylamino)methyl]-2-furyl]quinazolin-4-amine with CAS number 231277-92-2. Said compound also acts as a tyrosine kinase inhibitor.

As it is used herein, "HKI-272" or neratinib refers to the compound (2E)-N-[4-[[3-chloro-4-[(pyridin-2-yl)methoxy]phenyl]amino]-3-cyano-7-ethoxyquinolin-6-yl]-4-(dimethylamino)but-2-enamide with CAS number 698387-09-6. Said compound also acts as a tyrosine kinase inhibitor.

As it is used herein, "afatinib" or BIBW 2992 refers to the compound *N*-[4-[(3-chloro-4-fluorophenyl)amino]-7-[[(3*S*)-tetrahydro-3-furanyl]oxy]-6-quinazolinyl]-4(dimethylamino)-2-butenamide with CAS number 850140-72-6.

As it is used herein, "gefitinib" refers to the compound N-(3-chloro-4-fluoro-phenyl)-7-methoxy-6-(3-morpholin-4-ylpropoxy)quinazolin-4-amine with CAS number 184475-35-2.

As it is used herein, "icotinib" or BPI-2009H refers to the compound (1,4,7,10)Tetraoxacyclododecino(2,3-g)quinazolin-4-amine with CAS number 610798-31-7.

As it is used herein, "HDAC inhibitor" refers to a compound inhibiting the activity of histone deacetylase (HDAC), an enzyme involved in the removal of acetyl groups from the lysine residues of histones and in humans, the HDAC1 isoform corresponds to the protein with Uniprot database accession number Q13547 as of April 17, 2013. HDAC inhibitors also have effects on histone-free proteins which are related to the acetylation process, HSP90 being among them.

To find out if a compound is an HDAC inhibitor, it is possible to use any assay known in the state of the art for analyzing histone deacetylase activity, such as the commercial kit EpiQuick HDAC Activity/Inhibition Assay Kit of Epigentek, such that if the compound to be assayed inhibits histone deacetylase activity, said compound is an HDAC inhibitor.

In a particular embodiment of the present invention, the HDAC inhibitor is selected from the group consisting of vorinostat and panobinostat.

As it is used herein, "vorinostat" refers to the compound *N*-hydroxy-*N*'-phenyl-octanediamide with CAS number 149647-78-9.

As it is used herein, "panobinostat" or LBH-589 refers to the compound (2E)-*N*-hydroxy-3-[4-({[2-(2-methyl-1*H*-indol-3-yl)ethyl]amino}methyl)phenyl]acrylamide with CAS number 404950-80-7.

As it is used herein, "PARP inhibitor" refers to a compound inhibiting the activity of poly(ADP-ribose)polymerase (PARP), an enzyme involved in DNA repair and programmed cell death and in humans, PARP1 corresponds to the sequence with Uniprot database accession number P09874 as of April 17, 2013.

To find out if a compound is a PARP inhibitor, any method known in the state of the art for analyzing DNA repair and apoptosis can be used, such as the commercial kit Universal PARP Assay Kits of Trevigen®.

In a particular embodiment of the present invention, the PARP inhibitor is selected from the group consisting of iniparib, olaparib and veliparib.

As it is used herein, "iniparib" or BSI-201 refers to 4-iodo-3-nitrobenzamide with CAS number 16003-66-7.

As it is used herein, "olaparib" or AZD-2281 refers to 4-[(3-[(4-cyclopropylcarbonyl)piperazin-4-yl]carbonyl)-4-fluorophenyl]methyl(2H)phthalazin-1-one with CAS number 763113-22-0.

As it is used herein, "veliparib" or ABT-888 refers to the compound 2-((*R*)-2-methylpyrrolidin-2-yl)-1*H*-benzimidazole-4-carboxamide with CAS number 912444-00-9.

As it is used herein, "BRAF inhibitor" is understood as a compound inhibiting the activity of BARF which, in humans, encodes the protein with Uniprot database accession number P15056 as of April 17, 2013. The protein is member of the Raf kinase family and plays a role in the regulation of MAP kinase/ERK signaling pathways affecting cell division, differentiation and secretion.

To find out if a compound is a BRAF inhibitor, any method known in the state of the art can be used, such as the commercial kit B-Raf kinase assay kit of Millipore, based on the detection of MEK1 phosphorylation.

In a particular embodiment, the BRAF inhibitor is vemurafenib. As it is used herein, "vemurafenib" refers to the compound N-(3-(5-(4-chlorophenyl)-1H-pyrrolo[2,3-b]pyridine-3-carbonyl)-2,4-difluorophenyl)propane-1-sulfonamide with CAS number 1029872-54-5.

As it is used herein, "tyrosine kinase inhibitor" refers to a compound inhibiting the activity of a tyrosine kinase protein, an enzyme capable of transferring a phosphate group to a tyrosine residue of a protein.

To find out if a compound is a tyrosine kinase inhibitor, any method known in the state of the art can be used, such as those described in Hawes BE. et al., Curr Protoc Pharmacol. 2001 May; Chapter 3: Unit 3.5., or the commercial kit Tyrosine Kinase Assay Kit, Colorimetric Detection of Merck Millipore.

In a particular embodiment, the tyrosine kinase inhibitor is selected from the group consisting of sunitinib, sorafenib and pazopanib.

As it is used herein, "sunitinib" SU11248 refers to the compound *N*-[2-(diethylamino)ethyl]-5-[(Z)-(5-fluoro-1,2-dihydro-2-oxo-3*H*-indol-3-ylidine)methyl]-2,4-dimethyl-1*H*-pyrrole-3-carboxamide with CAS number 341031-54-7.

As it is used herein, "sorafenib" refers to the compound 4-[4-({[4-chloro-3-(trifluoromethyl)phenyl]carbamoyl}amino)phenoxy]-N-methylpyridine-2-carboxamide with CAS number 284461-73-0.

As it is used herein, "pazopanib" refers to the compound 5-[[4-[(2,3-dimethyl-2H-indazol-6-yl)methylamino]-2-pyrimidinyl]amino]-2-methylbenzolsulfonamide with CAS number 444731-52-6.

<u>Medical uses and compositions of the invention</u>

In another aspect, the invention relates to the use of an agent suitable for the treatment of brain metastasis for manufacturing a medicinal product for the treatment or prevention of brain metastasis in a subject who has increased levels of GRP94 with respect to a reference value and has been treated with a taxane.

Alternatively, the invention relates to an agent suitable for the treatment of brain metastasis for the treatment or prevention of brain metastasis in a subject who has increased levels of GRP94 with respect to a reference value and has been treated with a taxane.

In another aspect, the invention relates to the use of a taxane for manufacturing a medicinal product for the treatment of brain metastasis in a subject who has decreased levels of GRP94 with respect to a reference value.

Alternatively, the invention relates to a taxane for use in the treatment of brain metastasis in a subject who has decreased levels of GRP94 with respect to a reference value.

The manner in which a person skilled in the art can determine if a subject has increased or decreased levels of GRP94 with respect to a reference value has been described previously in relation to the methods of the invention.

As it is used herein, the term "treatment" refers to any type of therapy which aims at terminating, preventing, improving or reducing susceptibility to a clinical condition as described herein. In a preferred embodiment, the term treatment relates to prophylactic treatment (i.e., a therapy to reduce susceptibility to a clinical condition) of a disorder or a condition. Therefore, "treatment," "treating," and equivalent terms refer to obtaining a desired pharmacological or physiological effect, covering any treatment of a pathological condition or disorder in a mammal, including a human being. The effect may be prophylactic in terms of completely or partially preventing a disorder and/or adverse effect attributable to the disorder. In other words, "treatment" includes (1) preventing the disease from occurring or recurring in a subject, (2) inhibiting the disease, for example, by stopping its development, (3) interrupting or terminating the disorder or at least symptoms associated therewith, so the patient no longer suffers the disease or its symptoms, for example, causing regression of the disease or its symptoms by means of restoring or repairing a lost, missing or defective function, or stimulating an inefficient process, or (4) relieving, alleviating, or improving the disease or symptoms associated therewith, where improving is used in a broad sense to refer to at least a reduction in the magnitude of a parameter, such as inflammation, pain, or immune deficiency.

The terms "taxane" and "agent suitable for the treatment of metastasis" including the different agents have been described previously in relation to the methods of the invention.

In another aspect, the invention relates to a composition, composition of the invention, comprising a taxane and a GRP94 inhibitor and/or an FN14 inhibitor, wherein if the taxane is docetaxel, then the FN14 inhibitor is not the compound of formula (I) of Table 2.

In a particular embodiment, the GRP94 inhibitor is selected from Table 1.

| Table 1: GRP94 inhibitors suitable for use in the present invention | |
|---|---|
| I | Geldanamycin, benzoquinone ansamycin according to formula 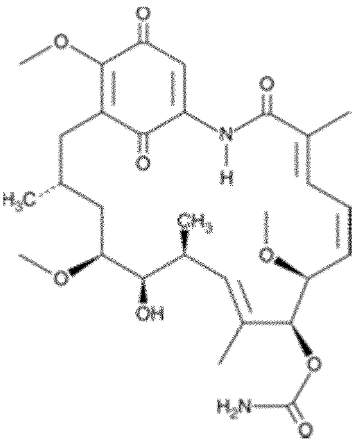 as described in Lawson B et al., J Cell Physiol, 1998 Feb;174(2):170-8. |
| II | Geldanamycin analogs containing a derivatization in position 17 such as<br>- 17-AAG (17-N-allylamino-17-demethoxygeldanamycin), tanespimycin according to formula 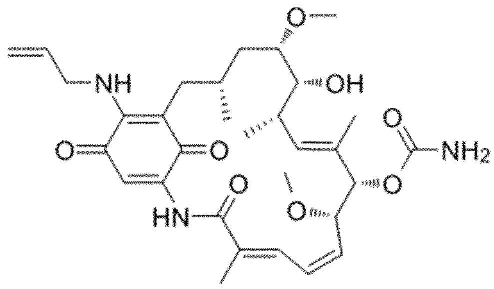<br>- 17-DMAG, alvespimycin, (17-dimethylaminoethylamino- 17-demethoxygeldanamycin) according to formula 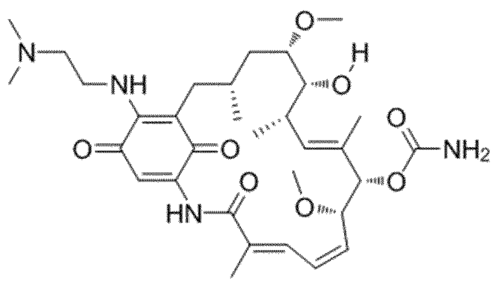 |
| III | "Compound 2" as described in Duerfeldt AS et al., J Am Chem Soc. June 13, 2012;134(23):9796-804. |
| IV | NVP-HSP990 according to formula |

(continued)

| Table 1: GRP94 inhibitors suitable for use in the present invention | |
|---|---|
| | 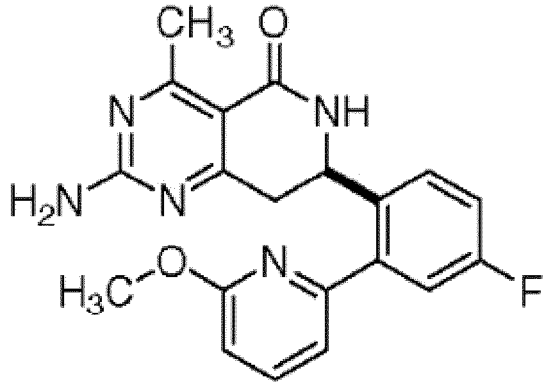<br>as described in Menezes DL et al., Mol Cancer Ther. Mar 2012;11(3):730-9 |
| V | IPI-504, Retaspimycin hydrochloride according to formula<br>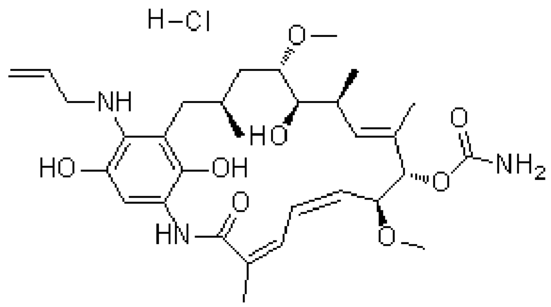<br>as described in Sequist Lecia V et al., JCO November 20, 2010 |
| VI | Molecules containing resorcinol such as<br>- NVP-AUY922 according to formula<br>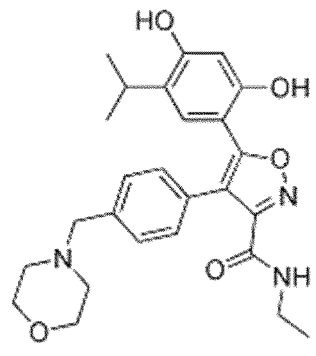<br>as described in Eccles SA, Cancer Res. Apr 15, 2008; 68 (8) :2850-60<br>- KW-2478 according to formula<br>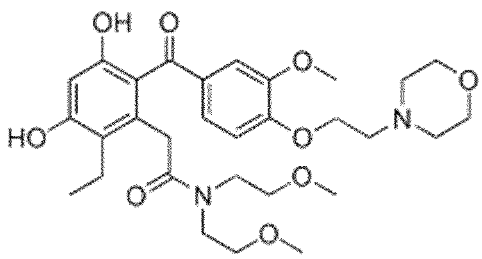<br>as described in Nakashima T et al., Clin Cancer Res. May 15, 2010;16(10):2792-802<br>- STA-9090, HY-15205, ganetespib, according to the formula |

(continued)

| Table 1: GRP94 inhibitors suitable for use in the present invention | |
|---|---|
| | 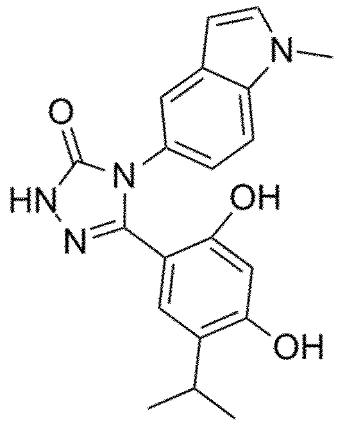<br>as described in Wang Y. et al., Curr Opin Investig Drugs. Dec 2010;11(12):1466-76. |
| VII | Molecules with purine backbone such as<br>- BIIB-021 Synonyms CNF2024; BIIB 021;CNF 2024;CNF- 2024, according to formula<br>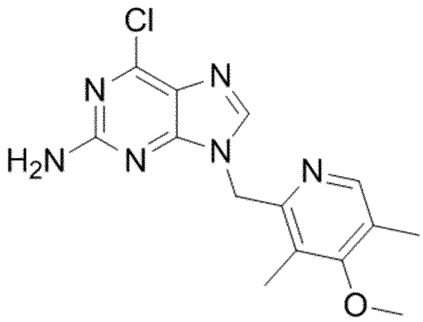<br>- PU-H71 according to formula<br>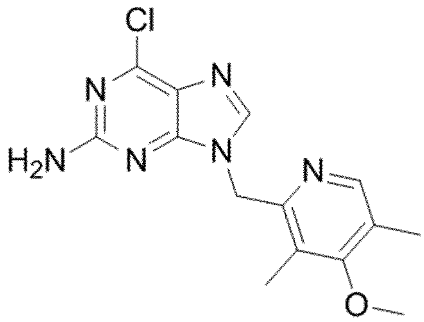<br>as described in Gallerne C. et al., Biochim Biophys Acta. June 2013;1833(6):<br>- MPC-3100 according to formula |

(continued)

| Table 1: GRP94 inhibitors suitable for use in the present invention | |
|---|---|
| | 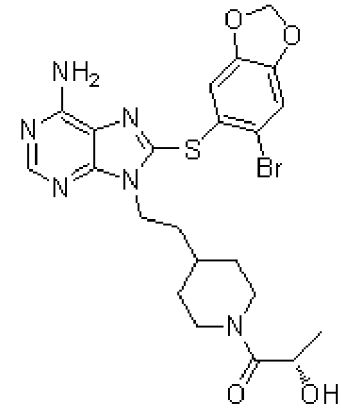 |
| | - MPC-0767 according to formula 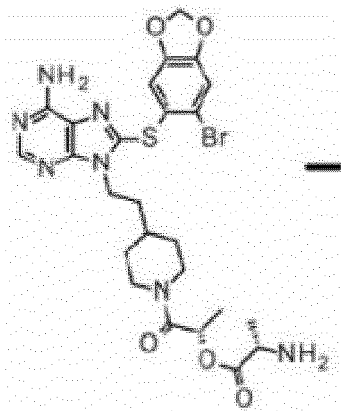 - AT13387 according to formula 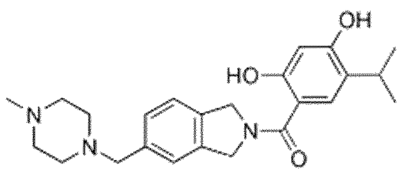 |
| VIII | 4,5 diarylisoxazole |
| IX | BIIB-028 |
| X | SNX-5422, mesylate according to formula 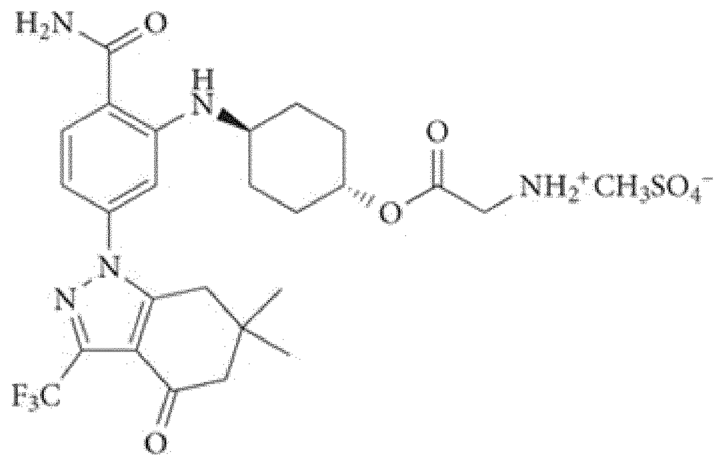 |

(continued)

| Table 1: GRP94 inhibitors suitable for use in the present invention | |
|---|---|
| XI | IPI-493 according to formula 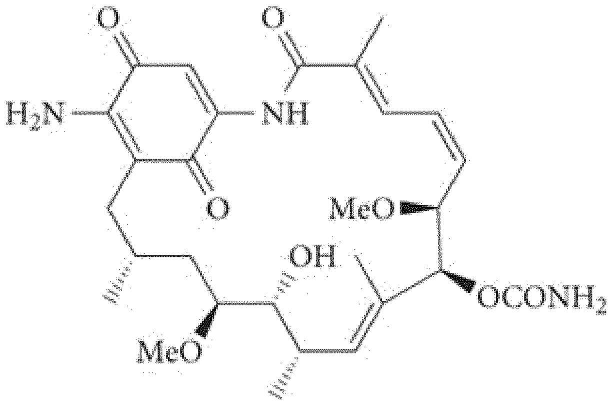 |
| XII | XL-888 according to formula 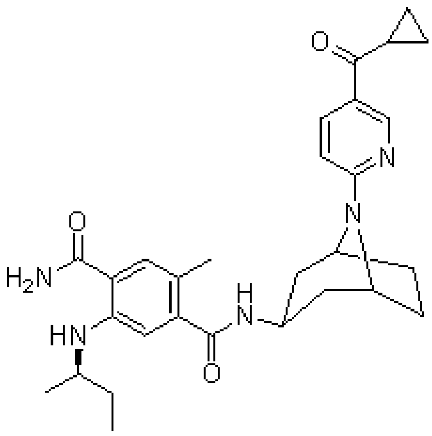 |
| XIII | Debio 0932 according to formula 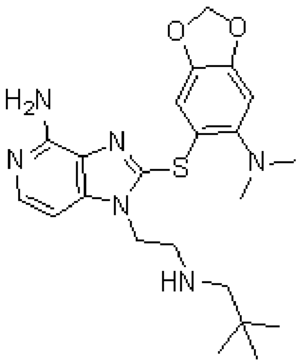 |
| XIV | NVP-HSP990 according to formula 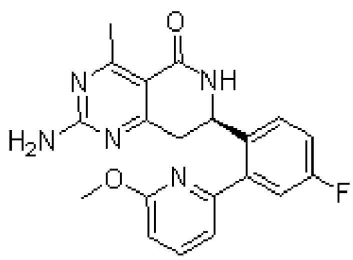 |

(continued)

| Table 1: GRP94 inhibitors suitable for use in the present invention | |
|---|---|
| XV | Generic ATPase inhibitors such as *5-N*-ethylcarboxamidoadenosine (NECA) |
| XVI | Inhibitory antibodies capable of binding specifically to GRP94 and inhibiting its activity |
| XVII | An interfering RNA |
| XVIII | A DNA enzyme or ribozyme specific for the GRP94 sequence, such as the ribozyme described in Little E. et al., J Biol Chem. Apr 21, 1995;270(16):9526-34 |
| XIX | An antisense oligonucleotide specific for the GRP94 sequence |
| XX | Overexpression of CYPE1 as described in Dey A. et al., Arch Biochem Biophys. Mar 15, 2006;447(2):155-66. |

In a particular embodiment, the FN14 inhibitor is selected from Table 2.

| Table 2: FN14 inhibitors suitable for use in the present invention | |
|---|---|
| I | Thalidomide derivatives such as the compound of formula (I)<br>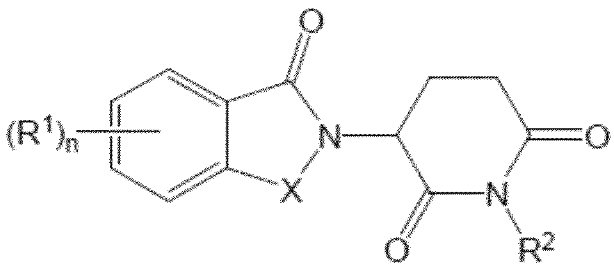<br>(I)<br>wherein<br>X is selected from $-CH_2-$ and -C(O);<br>$R^1$ is independently selected from halogen, $C_1$-$C_6$ alkyl, $C_1$-$C_6$ haloalkyl and NR'R'', wherein R' and R'' are independently selected from H and $C_1$-$C_6$ alkyl;<br>$R^2$ is selected from H and $C_1$-$C_6$ alkyl; and N is a integer selected from 0, 1, 2, 3 and 4;<br>For example, the compound with formula<br>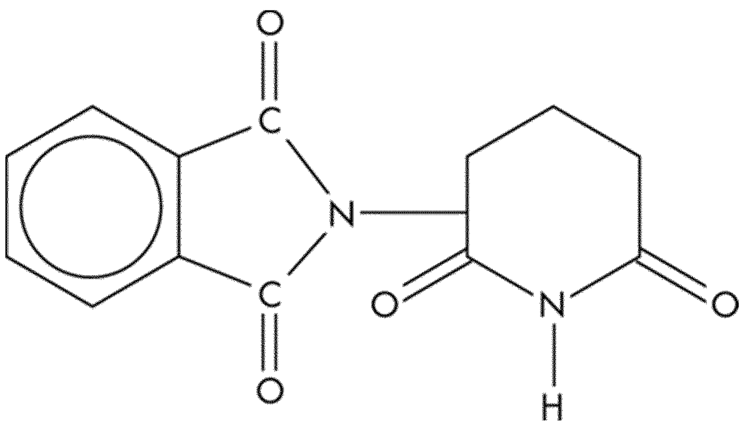<br>Lenalidomide according to formula |

(continued)

| Table 2: FN14 inhibitors suitable for use in the present invention | |
|---|---|
| | 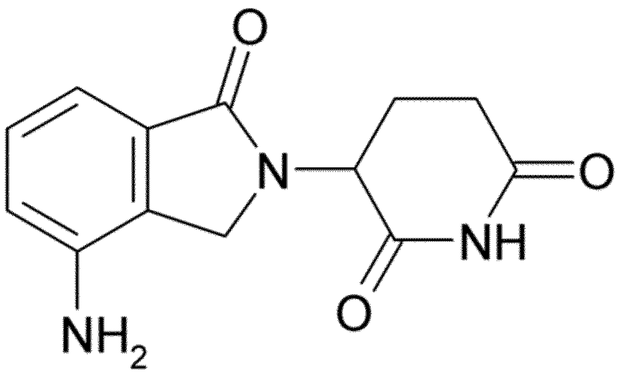 |
| II | Inhibitors of the inhibitors of apoptosis (IAPs), i.e., compounds inhibiting the activity of one or more IAPs, such as<br>- the compounds described in WO 2012052758 A1<br>- IAP-BIR2 antagonists, IAP-BIR3 antagonists or IAP-BIR3/BIR2 antagonists as described in Mannhold R. et al., Drug Discovery Today, Volume 15, Issues 5-6, March 2010, Pages 210-219<br>- Compounds with general formula (I)<br>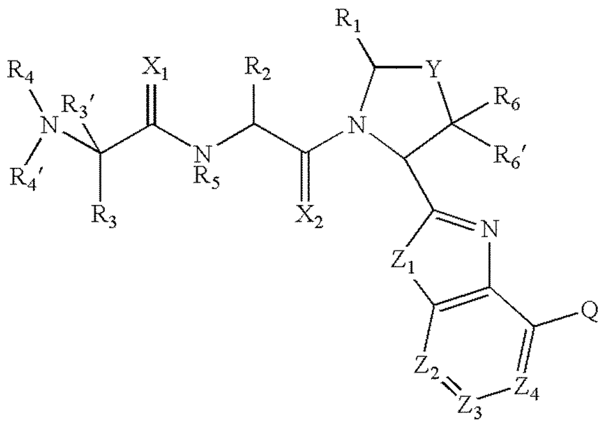<br>as described in patent US 8247557 B2<br>- Compound C and compound D as described in WO 2005074989 A2<br>- Compounds with general formula |
| | 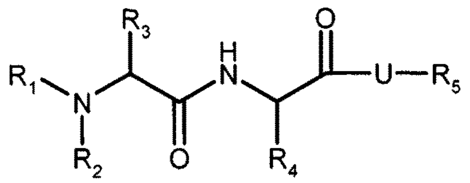 (I)<br>as described in WO 2008067280 A2 |
| III | Agents blocking the interaction of Tweak and FN14, among them, Anti-Tweak antibodies as described in Yumin Xia et al., Clinical Immunology Volume 145, Issue 2, November 2012, Pages 108-121 |
| IV | Atorvastatin as described in Muñoz-García B. et al, Stroke.2006;37: 2044-2053 |
| V | Geranylgeranyl transferase inhibitors such as GGTI described in Muñoz-García B. et al., Stroke.2006;37: 2044-2053 |
| VI | Rac and Rho inhibitors such as toxin B as described in Muñoz-García B. et al., Stroke.2006;37: 2044-2053 |
| VII | Rho inhibitors such as exoenzyme C3 as described in Muñoz-García B. et al., Stroke.2006;37: 2044-2053 |

(continued)

| Table 2: FN14 inhibitors suitable for use in the present invention | |
|---|---|
| VIII | Rho kinase inhibitors such as Y-27632 described in Muñoz-García B. et al., Stroke.2006;37: 2044-2053 |
| IX | FN14-binding proteins such as those described in WO 2013026099 A1 |
| X | Inhibitory antibodies capable of binding specifically to FN14 and inhibiting its activity. |
| XI | An interfering RNA |
| XII | A DNA enzyme or ribozyme specific for the FN14 sequence |
| XIII | An antisense oligonucleotide specific for the FN14 sequence |

In the context of the present invention, "inhibitory antibody" is understand as any antibody which is capable of binding specifically to the grp94 protein or to the fn14 protein and inhibiting one or more of the functions of said proteins. The antibodies can be prepared using any of the methods which are known for the person skilled in the art. Therefore, polyclonal antibodies can be prepared by means of immunizing an animal with the protein to be inhibited. Monoclonal antibodies can be prepared using the method described by Kohler, Milstein et al. (Nature, 1975, 256: 495). Suitable antibodies in the context of the present invention include whole antibodies comprising a variable antigen-binding region and a constant region, "Fab", "F(ab')2" and "Fab'", Fv, scFv fragments, diabodies and bispecific antibodies. Once antibodies capable of binding to the grp94 protein are identified, those capable of inhibiting the activity of this protein will be selected using an inhibitory agent identification assay.

grp94 antibodies which can be used in the present invention are, for examples, those described in the table of application US2012148598. fn14 antibodies which can be used in the present invention are, for example, PDL192 and P4A8, or those described in EP 2294089 A2.

Small interfering RNAs or siRNAs are agents capable of inhibiting the expression of a target gene by means of RNA interference. A siRNA can be chemically synthesized, obtained by means of *in vitro* transcription or synthesized *in vivo* in the target cell.

Typically, siRNAs consist of double-stranded RNA between 15 and 40 nucleotides long and can contain a 3' and/or 5' protruding region of 1 to 6 nucleotides. The length of the protruding region is independent of the total length of the siRNA molecule. siRNAs act by means of degrading or silencing the target messenger after transcription.

The siRNAs of the invention are substantially homologous to the mRNA of the gene encoding GRP94 or to the gene sequence encoding said protein. "Substantially homologous" is understood as having a sequence which is sufficiently complementary or similar to the target mRNA such that the siRNA is capable of degrading the latter through RNA interference. The siRNAs suitable for causing said interference include siRNAs formed by RNA, as well as siRNAs containing different chemical modifications such as:

- siRNAs in which the bonds between nucleotides are different than those occurring in nature, such as phosphorothioate bonds
- conjugates of the RNA strand with a functional reagent, such as a fluorophore
- modifications of the ends of the RNA strands, particularly the 3' end, by means of modification with different hydroxyl functional groups in 2' position
- nucleotides with modified sugars such as O-alkylated residues in 2' position such as 2'-O-methylribose or 2'-O-fluororibose
- nucleotides with modified bases such as halogenated bases (for example, 5-bromouracil and 5-iodouracil), alkylated bases (for example, 7-methylguanosine)

Specific siRNAs for GRP94 include the siRNAs described in US20090186039 such as 5'-UGAUGU GGA UGG UACAGU A-3' (SEQ ID NO: 1), 5'-UACUGU ACC AUC CAC AUC A-3' (SEQ ID NO: 2), 5'-GAAGAA GCA UCU GAU UAC C-3' (SEQ ID NO: 3), 5'-GCCUUC CAA GCC GAA GUU A-3' (SEQ ID NO: 4), 5'-CCUUCC AAG CCG AAG UUA A-3' (SEQ ID NO: 5), 5'-UCUGGA AAU GAG GAA CUA A-3' (SEQ ID NO: 6), 5'-CCUUGG UAC CAU AGC CAA A-3' (SEQ ID NO: 7), 5'-GCAUCU GAU UAC CUV GAA OR-3' (SEQ ID NO: 8),5'-GGAGUC UGA CUC CAA UGA A-3' (SEQ ID NO: 9).

Specific siRNAs of FN14 include the siRNAs 5'-GGAUUCGGC UUG GUG UUG AUU3' (SEQ ID NO: 10); 5'-CGU CGUCCAUUCAUUCAUUUU-3' (SEQ ID NO: 11); 5'-GGACUGGGCUUAGAGUUCAUU-3' (SEQ ID NO: 12); and 5'-CUAAGGAACUGCAGCAUUUUU-3' (SEQ ID NO: 13).

On the other hand, the invention also contemplates the use of DNA enzymes to inhibit the expression of the GRP94 gene or FN14 gene. DNA enzymes incorporate some of the mechanistic features of both antisense and ribozyme

technologies. DNA enzymes are designed such that they recognize a particular target nucleic acid sequence similar to the antisense oligonucleotide, however, like the ribozyme they are catalytic and specifically cleave the target nucleic acid.

Ribozyme molecules designed for catalytically cleaving target mRNA transcripts to prevent the translation of the mRNAs encoding GRP94 or FN14, the activity of which is to be inhibited, can also be used. Ribozymes are enzymatic RNA molecules capable of catalyzing specific RNA cleaving (for a review, see Rossi, Current Biology 4: 469-471, 1994). The mechanism of ribozyme action involves specific hybridization of a ribozyme molecule sequence to a complementary target RNA followed by an endonucleolytic cleavage event. The composition of the ribozyme molecules preferably includes one or more sequences complementary to the target mRNA and the well-known sequence responsible for cleaving the mRNA or a functionally equivalent sequence (see, for example, US patent no. 5093246).

The ribozymes used in the present invention include hammerhead ribozymes and endoribonuclease RNA (hereinafter, "Cech-type ribozymes") (Zaug et al., Science 224:574-578, 1984).

As it is used herein, the term "antisense oligonucleotide" refers to a polynucleotide which is capable of hybridizing specifically with a specific DNA or RNA and includes DNA oligonucleotides, RNA oligonucleotides or chimeric mixtures, as well as single- or double-stranded oligonucleotides.

An additional aspect of the invention relates to the use of isolated "antisense" nucleic acids to inhibit expression, for example, for inhibiting transcription and/or translation of a nucleic acid encoding GRP94 or FN14 the activity of which is to be inhibited. The antisense nucleic acids can bind to the potential target by means of conventional base complementarity or, for example, in the case of binding to a double-stranded DNA, through specific interaction in the major groove of the double helix. Generally, these methods refer to a range of techniques generally used in the art and include any method which is based on the specific binding to oligonucleotide sequences.

An antisense construct of the present invention can be distributed, for example, as an expression plasmid which, when transcribed in the cell, produces RNA complementary to at least one unique part of the cellular mRNA encoding GRP94 or FN14. Alternatively, the antisense construct is an oligonucleotide probe generated *ex vivo* which, when introduced into the cell, inhibits gene expression by hybridizing with the mRNA and/or gene sequences of a target nucleic acid. Such oligonucleotide probes are preferably modified oligonucleotides which are resistant to endogenous nucleases, for example, exonucleases and/or endonucleases, and are therefore stable *in vivo.* Examples of nucleic acid molecules for use as antisense oligonucleotides are DNA analogs of phosphoramidate, phosphothionate and methylphosphonate (see also US patent nos. 5176996, 5264564 and 5256775). Additionally, general approaches for constructing oligomers useful in antisense therapy have been reviewed, for example, in Van der Krol et al., BioTechniques 6: 958-976, 1988; and Stein et al., Cancer Res 48: 2659-2668, 1988.

With respect to the antisense oligonucleotide, the oligodeoxyribonucleotide regions derived from the translation start site, for example, between -10 and +10 of the target gene, are preferred. Antisense approaches involve designing oligonucleotides (either DNA or RNA) that are complementary to the mRNA encoding the target polypeptide. Antisense oligonucleotides will bind to mRNA transcripts and prevent translation.

The oligonucleotides which are complementary to the 5' end of the mRNA, for example, the non-translated 5' sequence up to and including the AUG start codon, must function in the most efficient manner to inhibit translation. However, it has recently been shown that sequences complementary to the non-translated 3' sequences of the mRNA are also efficient for inhibiting mRNA translation (Wagner, Nature 372: 333, 1994). Therefore, complementary oligonucleotides could be used in the non-translated 5' or 3' regions, the non-coding regions of a gene in an antisense approach to inhibit the translation of that mRNA. The oligonucleotides complementary to the non-translated 5' region of the mRNA must include the complement of the AUG start codon. The oligonucleotides complementary to the coding region of the mRNA are less efficient translation inhibitors, but they could also be used according to the invention. If they are designed to hybridize with the 5' region, 3' region or the coding region of the mRNA, antisense nucleic acids must be at least six nucleotides long and preferably less than about 100, and more preferably less than about 50, 25, 17 or 10, nucleotides long.

Preferably, *in vitro* studies are first performed to quantify the capacity of the antisense oligonucleotides for inhibiting gene expression. Preferably, these studies use controls which distinguish between antisense gene inhibition and non-specific biological effects of oligonucleotides. Also preferably, these studies compare the levels of target RNA or protein with that of an internal RNA or protein control. The results obtained using antisense oligonucleotides can be compared with those obtained using a control oligonucleotide. Preferably, the control oligonucleotide is about the same length as the oligonucleotide to be assayed, and the oligonucleotide sequence preferably differs from the antisense sequence by no more than what is deemed necessary to prevent specific hybridization to the target sequence.

The oligonucleotide can be modified in the base group, the sugar group or the phosphate backbone, for example, to improve the stability of the molecule, its hybridization capacity, etc. The oligonucleotide can include other bound groups, such as peptides (for example, for directing them to the receptors of the host cells) or agents for facilitating transport through the cell membrane (see, for example, Letsinger et al., Proc. Natl. Acad. Sci. U.S.A. 86: 6553-6556, 1989; Lemaitre et al., Proc. Natl. Acad. Sci. 84: 648-652, 1987; PCT Publication No. WO88/09810) or the blood-brain barrier (see, for example, PCT publication No. WO89/10134), intercalating agents (see, for example, Zon, Pharm. Res. 5: 539-549, 1988). For this purpose, the oligonucleotide can be conjugated to another molecule, for example, a peptide,

a transporting agent, hybridization-triggered cleaving agent, etc.

Antisense oligonucleotides may comprise at least one modified base group. The antisense oligonucleotide can also comprise at least one modified sugar group selected from the group including, but not limited to, arabinose, 2-fluoroarabinose, xylulose and hexose. The antisense oligonucleotide can contain a backbone similar to a neutral peptide. Such molecules are referred to as peptide nucleic acid (PNA) oligomers and are described, for example, in Perry-O'Keefe et al., Proc. Natl. Acad. Sci. U.S.A. 93: 14670, 1996, and in Eglom et al., Nature 365: 566, 1993.

In yet another embodiment, the antisense oligonucleotide comprises at least one modified phosphate backbone. In still another embodiment, the antisense oligonucleotide is an alpha-anomeric oligonucleotide.

While antisense oligonucleotides complementary to the coding region of the target mRNA sequence can be used, those complementary to the transcribed non-translated region can also be used.

In some cases, it may be difficult to reach sufficient intracellular concentrations of the antisense oligonucleotide to suppress endogenous mRNA translation. Therefore, a preferred approach uses a recombinant DNA construct in which the antisense oligonucleotide is placed under the control of a strong pol III or pol II promoter.

Alternatively, the expression of the target gene can be reduced by directing deoxyribonucleotide sequences complementary to the gene regulating region (i.e., the promoter and/or enhancers) to form triple helix structures preventing gene transcription in target cells in the body (see in general, Helene, Anticancer Drug Des. 6(6): 569-84, 1991). In certain embodiments, the antisense oligonucleotides are antisense morpholines.

In another aspect, the invention refers to the use of a composition of the invention for manufacturing a medicinal product for the treatment of brain metastasis.

The compositions of the invention for manufacturing a medicinal product for use according to the present invention can be administered by any suitable route of administration, for example, oral, sublingual, topical, rectal or parenteral, including subcutaneous, intraperitoneal, intradermal, intramuscular, intravenous, intravascular, intratumoral, intracranial, intrathecal, intrasplenic, subretinal and mucosal routes.

In a particular embodiment of the invention, the medicinal product is administered through the intrathecal and/or intratumoral route.

As understood by the person skilled in the art, the direct administration of the composition of the invention to the site which is to benefit from said administration can sometimes be advantageous. Therefore, direct administration of the composition of the invention to the desired organ or tissue can be achieved by direct administration (e.g., by injection, etc.) on the outer surface of the affected organ or tissue by means of inserting a suitable device, e.g., a cannula or pump suitable for administration, by arterial or renal perfusion (including backward flow mechanisms) or by other means mentioned in this description or known in the art. In the case of direct administration in the brain, it is common to use a catheter in the ventricular system through intrathecal puncture or pump for administration to avoid the blood-brain barrier.

In any case, preferably if the composition of the invention is to be administered through means other than intracranial means, the formulation of said compound must allow going through the blood-brain barrier.

Molecule entry in the brain is efficiently controlled by the blood-brain barrier, such that only small lipophilic molecules are capable of going through it by passive diffusion. Therefore, for use according to the invention, the composition of the invention should be capable of going through the blood-brain barrier in sufficient amounts so as to have a desired pharmacological effect.

The criteria that a compound must have in order to go through the blood-brain barrier in a passive manner are disclosed, for example, in Table 2.1 of the document by Gynther M. Publications of the University of Eastern Finland, Dissertations in Health Sciences, 2010.

There are various methods known in the state of the art which allow determining and estimating the permeability of different drugs in the brain. Among them, *in vitro* models such as in isolated brain capillaries, brain capillary endothelial cell cultures and immortalized artificial membranes, as described in Lundquist and Renftel Vascul Pharmacol 38: 355-364, 2002, stand out. It is also possible to use astrocytes as a model, as described in Li et *al.,* 2010 or a hollow-fiber cartridge model as described in Neuhaus et *al.,* 2006. Other models are the rat brain perfusion model as described in (Takasato et al., Am J Physiol 247: H484-493, 1984) or brain microdialysis (de Lange et al., Adv Drug Deliv Rev 36: 211-227, 1999).

Antisense oligonucleotides can include other groups bound thereto, such as the agents described in WO89/10134, in order to go through the blood-brain barrier.

Alternatively, the compound is preferably fat soluble or it is modified so that it is fat soluble, for example, by blocking the hydroxyl, carboxyl and primary amine groups. Alternatively, it is possible to administer in an intra-arterial manner a hypertonic solution such that it opens the blood-brain barrier temporarily and allows the passage of hydrophilic drugs. Alternatively, it is possible to use some transporters present in the endothelium of the brain capillaries such as LAT and Glut1, modifying the drugs such that they are recognized by said transporters as described in Rautio et al., Aaps J 10: 92-102 2008 and in Gynther M. Publications of the University of Eastern Finland, Dissertations in Health Sciences, 2010.

In a particular embodiment, the compositions of the invention are in encapsulation systems. A variety of suitable

encapsulation systems is known in the art ("Microcapsules and nanoparticles in Medicine and Pharmacy", edited by Doubrow, M., CRC Press, Boca Raton, 1992; Mathiowitz and Langer J. Control. Release 5:13, 1987; Mathiowitz et al. Reactive Polymers 6:275, 1987; Mathiowitz et al. J. Appl. Polymer Sci. 35:755, 1988; Langer Ace. Chem. Res. 33:94,2000; Langer J. Control. Release 62:7,1999; Uhrich et al. Chem. Rev. 99:3181,1999; Zhou et al. J. Control. Release 75:27, 2001; and Hanes et al. Pharm. Biotechnol. 6:389,1995). Among these systems, the preparation of colloidal suspensions, such as liposomes, microemulsions, nanospheres and nanoparticles, based on the formation of an oily stage in which the active ingredient is incorporated dispersed in an aqueous stage, as a result of using one or several surface active agents, essentially stands out. Liposomes can be prepared using conventional techniques including sonication, dialysis with chelates, homogenization, solvent infusion coupled with extrusion, extrusion by freezing-thawing, microemulsification, etc. The reagents used for cross-linking a liposome or another lipid-containing agent comprise a phospholipid derivative for anchoring to an end of the cross-link in the lipid layer and a reactive group at the other end to provide a point for binding to the target biomolecule.

Liposomes can comprise one or more moieties which are selectively transported to specific cells or organs, thus enhancing target drug administration (see, e.g., V.V. Ranade (1989) J. Clin. Pharmacol. 29:685). Examples of site-directed moieties include folate or biotin (see, e.g., US patent 5,416,016 of Low et al.); mannosides (Umezawa et al., (1988) Biochem. Biophys. Res. Comman. 153:1038); antibodies (P.G. Bloeman et al. (1995) FEBS Lett. 357:140; M. Owais et al. (1995) Antimicrob. Agents Chemother. 39:180); surface-active protein A receptor (Briscoe et al. (1995) Am. J. Physiol. 1233:134); page 120 (Schreier et al. (1994) J. Biol. Chem. 269:9090); also see K. Keinanen; MX. Laukkanen (1994) FEBS Lett. 346:123; J.J. Killion; I.J. Fidler (1994) Immunomethods 4:273.

For application in therapy, the compositions of the invention are preferably in an acceptably or substantially pure form, i.e., the compositions of the invention have a pharmaceutically acceptable level of purity excluding the pharmaceutically acceptable excipients and without including material considered toxic at normal dosage levels. The levels of purity for the compounds preferably exceed 50%, more preferably exceed 70%, and more preferably exceed 90%. In a preferred embodiment, they exceed 95%.

The pharmaceutical compositions of the invention can be administered in doses of less than 10 mg per kilogram of body weight, preferably less than 5, 2, 1, 0.5, 0.1, 0.05, 0.01, 0.005, 0.001, 0.0005, 0.0001, 0.00005 or 0.00001 mg per kg of body weight. The unit dose can be administered by injection, by inhalation or by topical administration.

The dose depends on the severity and response of the condition to be treated and it may vary between several days and months or until the condition subsides. The optimal dosage can be determined by periodically measuring the concentrations of the agent in the body of the patient. The optimal dose can be determined from the EC50 values obtained by means of previous *in vitro* or *in vivo* assays in animal models. The unit dose can be administered once a day or less than once a day, preferably less than once every 2, 4, 8 or 30 days. Alternatively, it is possible to administer a starting dose followed by one or several maintenance doses, generally of a lesser amount than the starting dose. The maintenance regimen may involve treating the patient with a dose ranging between 0.01 $\mu$g and 1.4 mg/kg of body weight per day, for example 10, 1, 0.1, 0.01, 0.001, or 0.00001 mg per kg of body weight per day. Maintenance doses are preferably administered once every 5, 10 or 30 days at most. Treatment must be continued for a time that will vary according to the type of disorder the patient suffers, the severity thereof and the condition of the patient. After treatment, the progress of the patient must be monitored to determine if the dose should be increased in the event that the disease does not respond to the treatment, or the dose is reduced if an improvement of the disease is observed or if unwanted side effects are observed.

The invention is now described in detail by means of the following examples which must only be considered as merely illustrative and non-limiting of the scope of the invention.

## Examples

### Example 1- Effect of the inhibition of the expression of GRP94

The 435BR cells metastasizing to the brain, derived from MDA-MB 435 breast cancer cells, were used for selecting a highly brain metastatic phenotype when injected in the left ventricle (l.v.) and/or in the internal carotid artery (i.c.). After infecting them with a retrovirus containing the green protein and luciferase gene (eGFP-CMV/Luc), 5 successive passes were made *in vivo*/*in vitro* in which the cells were injected in the l.v. of immunosuppressed mice and when they developed metastasis, primary cultures were made to be injected again into the test animal (Figure 1). The cells from the fifth pass, BRV5, were injected into the internal carotid artery and a new variant BRV5CA1 causing death of all mice due to brain metastasis was obtained (Martinez-Aranda A., Int. J. Mol. Sci.2013, 14(4), 8306-8327).

The cells metastasizing to the brain are characterized by their high expression level of GRP94, so GRP94 was silenced to study the cause-effect of this protein on the process of brain metastasis.

The brain metastatic variant BRV5CA1 was transformed with 5 commercial shRNAs for GRP94 of Sigma-Aldrich (MISSION® shRNA Bacterial Glycerol Stock, Sigma-Aldrich). Clones were obtained from the 5 lines generated

by limiting dilution. Those clones having silenced GRP94 were selected by means of Western blot, PCR and immunofluorescence.

To study the relationship between the expression level of GRP94 and the response to taxol of breast cancer cells metastasizing to the brain, three sublines were used: Clones: 424-2 and 424-8 (silenced GRP94 [shGRP94]) and a Pool (non-silenced GRP94), from the metastatic variant BRV5Ca1.

For the cytotoxicity assay, each cell type was seeded in triplicate in a 96-well plate (*costar® 3596; Corning Incorporated)* at a density of 6000 cells/well in 50 μL of complete medium (*DMEM/F12 GTBCO®* with serum). Twenty-four hours after seeding the cells, they were treated with taxotere (docetaxel > 99%; LC Laboratories (www.LCLabs.com); Stock 200 mg/ml), at different concentrations in triplicate: 10 - 5 - 2.5 - 1 and 0 ng/ml, in a volume of 50 μL/well. Seventy-two hours after the start of the treatment, a cell viability assay MTT was carried out in which survival expressed as a percentage (%) was analyzed (Figure 2). Before the assay (10 minutes before), a negative control was included by treating the cells with Triton X-100 (Sigma-Aldrich, CAS Number: 9002-93-1) in a volume of 30 μL/well, for obtaining the maximum mortality value.

After this time, the medium was removed from the wells and 50 μL of MTT [3-(4,5-dimethylthiazol-2-yl)-2,5-diphenyltetrazolium bromide; Sigma-Aldrich) were added per well, at a concentration of 5 mg MTT/ml of medium without serum. The cells were incubated at 37°C for about 2-4 hours in the dark. Then MTT was removed and 100 μL of 100% DMSO were added per well *(Sigma Aldrich*). It was left to stir for 10-15 minutes (in the dark) and was then analyzed in the microplate spectrophotometer (*PowerWave™ XS; BioTek*) at a wavelength of 540 nm.

Absorbance values (OD) were obtained in triplicate for each of the treatment concentrations established. The calculated mean was expressed in reference to the control thereof (0 ng/ml), considered as the maximum survival value (100%).

$$Survival\ (\%) = \frac{Treatment\ absorbance}{Control\ absorbance} * 100$$

As seen in Figure 2, cells 424-2 and 424-8 have lower survival after treatment with taxol.

Example 2- Effect of taxotere on tumor growth

A xenotransplant of breast cancer metastasizing to the brain provided by Dr. Eva Gonzalez was used to induce breast tumors in immunosuppressed Athymic Nude-Foxn1nu mice weighing 22-28 grams acquired from Charles-River Laboratories (Wilmington, MA) and to establish groups that are treated or not treated with taxotere following a previously established standard protocol of 15 mg/kg/day every 4 days for two weeks (Bin Gu et al., Carcinogenesis, 2004). The 2 $mm^3$ tumor fragment was implanted in the inguinal breast of female mice and 10 days were waited out for the graft to progress. After evaluating the volume of the tumor, treatment with taxotere was started, considering in each case a control group which was treated with the drug carrier.

The expression of the grp94 protein in the control tumors and in the treated tumors was examined to study the possible secondary modifications of expression with respect to treatment.

Figure 3 shows the progression of the means of the response variable (normalized volume divided by the initial volume at the start of treatment) from the starting day for each of the groups. This graph shows how the time variable has a very positive and significant effect on tumor growth in mice treated with the carrier (control). Although the tumors treated with taxotere show a tendency to grow less than that of the controls (p=0.003), they progress over time. The interaction between time and treatment with taxotere is negative and significant (p=0.003), indicating that the tumors treated with taxotere have a tendency to grow less than that of the control.

Example 3- Relationship of the expression of GRP94 and/or FN14 with the risk of suffering brain metastasis

A multicenter study including 318 tumor samples from breast cancer patients has been conducted and the expression of GRP94 and FN14 in tissue embedded in paraffin originating from the Pathological Anatomy archive of three hospitals has been analyzed. In addition to these binary markers, the following co-variables have been taken into account: tumor size, smaller or bigger than 20 mm; histology grade, 1, 2 and 3; presence of affected nodes: 0 nodes +, 1-3 nodes +, 4 or more nodes +; estrogen receptors (ERs) and progesterone receptors (EPs): negative or positive; HER2: negative/faint or positive; adjuvant chemotherapy: taxanes or no taxanes and initial adjuvant hormone therapy: yes or no.

**[0162]** To check if the markers maintain their importance in the presence of these co-variables (all of them binary or with 3 categories, the first category being included by reference), multivariate models of each marker have been made including the effect of the co-variables (Figure 4). The sensitivity and specificity data is shown in Table 3.

Table 3. Sensitivity and specificity values obtained for each marker.

| Marker | Sensitivity | Specificity |
|---|---|---|
| HER2 | 24.68% | 89.55% |
| GRP94 | 65.06% | 57.83% |
| FN14 | 38.27%, | 89.43% |
| GRP94 + FN14 | 74.07%, | 51.98% |
| GRP94 + FN14 + HER2 | 72.37% | 53.49% |

Multivariate analysis indicates that the detection of the expression of GRP94 (AUC =0.61) alone and FN14 (AUC=0.64) alone is better for predicting the risk of brain metastasis compared with HER2 (AUC =0.57). The combination of GRP94 and FN14 is even better (AUC =0.69) (Figure 4D).

Brain metastasis survival curves have been calculated considering whether or not a patient has suffered brain metastasis, as an event in the moment in which this metastasis has been recorded. If a patient does not suffer metastasis, the patient is considered censured at the end of the follow-up period (Cox model). Figure 5 shows the survival curves considering that the patient has a tumor which overexpresses or does not overexpress FN14. The brain metastasis-free time of patients who have followed the therapeutic regimens with taxanes or others (which did not include taxane) is depicted. The expression of FN14 is significantly associated with disease-free survival according to patients who followed therapies with taxanes or without taxanes ($n = 37$; $p = 0.0475$).

Example 4- Lenalidomide inhibits the expression of FN14

Experimental tumors induced in the breast of immunosuppressed mice by implanting small biopsy fragments (2x2 mm) from patients with triple-negative breast carcinoma. An example of one of these grafts treated with lenalidomide (LND) with respect to the same graft treated with the carrier (control) is shown.

For expression studies, the immunohistochemistry technique with specific anti-FN14 antibodies (sc-27143, C-13, Santa Cruz Biotechnology) at a concentration of 1/3000 was used. Kidney and heart tissues were used as control tissues to view the expression in the cell membrane. The antigens were exposed by heating the preparations in a pressure cooker for 7 minutes in the suitable citrate buffer (Sanz et al., Am J Pathol, 179(2) :564-579, 2011).

The primary antibody was diluted in the Dako diluent buffer (Dako, Glostrup, Denmark; Carpinteria, CA): Tris buffer, pH 7.2, 15 mmol/l of $NaN_3$. The LSAB peroxidase system (Dako), including biotinylated secondary antibody, followed by conjugated streptavidin, was used along with the ultraView detection kit (Ventana Benchmark XT, Roche, Tucson, AZ). The tumors were classified in three categories considering staining intensity: negative, weakly positive and strongly positive. Only those which are strongly positive in more than 30% of the tumor cells were considered with the overexpressed protein.

Figure 6 shows inhibition of the expression of FN14 after treatment with lenalidomide.

Example 5- Therapeutic effect of an FN14 inhibitor

The therapeutic effect of lenalidomide was analyzed by treating the brain metastases of breast cancer induced by injecting BRV5CA1 cells labeled with the luciferase gene into female Athymic Nude-Foxn1nu mice (Martinez-Aranda A., Int. J. Mol. Sci.2013, 14(4), 8306-8327). The treatment groups N= 8 (lenalidomide 50 mg/kg/d on consecutive days) and the control group, in which carrier was injected, were established. The start of the treatment was established on day 14 after the graft and treatment was maintained for 15 days until the end of the experiment. The treatment was started on day 13 after induction and progression of the growth of metastasis was monitored *in vivo* by quantifying luciferase emission (photons per second) (Figure 7). After a week of treatment, the difference in growth of the metastasis between the control group and the treated group was significant.

## Claims

1. An *in vitro* method for predicting metastasis in a subject diagnosed with breast cancer and treated with a taxane comprising

    i) determining the expression level of the GRP94 gene and/or the expression level of the FN14 gene in a sample

from said subject, and
ii) comparing the expression level obtained in step i) with a reference value for each gene

wherein an increased expression level of GRP94 and/or an increased level of FN14 with respect to said reference value is indicative of a high risk of developing metastasis, or wherein a decreased expression level of GRP94 and/or a decreased level of FN14 with respect to said reference value is indicative of a low risk of developing metastasis.

**2.** An *in vitro* method for selecting breast cancer patients having a risk of developing metastasis after treatment with a taxane comprising

i) determining the expression level of the GRP94 gene and/or the expression level of the FN14 gene in a sample from said subject, and
ii) comparing the expression level obtained in step i) with a reference value for each gene

wherein a decreased expression level of GRP94 and/or a decreased level of FN14 with respect to said reference value is indicative that said patient has a low risk of developing metastasis after treatment with a taxane.

**3.** The method according to any of claims 1 or 2, wherein the metastasis is a brain metastasis.

**4.** An *in vitro* method for designing personalized therapy for a subject suffering breast cancer and being treated with a taxane comprising

i) determining the expression level of the GRP94 gene and/or the expression level of the FN14 gene in a sample from said subject, and
ii) comparing the expression level obtained in step i) with a reference value for each gene

wherein if the expression level of GRP94 and/or the level of FN14 is decreased with respect to said reference value, then said subject is not susceptible to be treated with an agent suitable for the treatment of brain metastasis, or wherein if the expression level of GRP94 and/or the level of FN14 is increased with respect to said reference value, then said subject is susceptible to receive treatment with an agent suitable for the treatment of brain metastasis.

**5.** The method according to any of claims 1 to 4, wherein the quantification of the expression level of the GRP94 gene and/or FN14 gene comprises quantifying the levels of protein encoded by said gene or of a variant thereof.

**6.** The method according to claim 5, wherein the levels of protein are quantified by means of immunohistochemistry, Western blot, ELISA or a protein array.

**7.** The method according to any of claims 1 to 4, wherein the quantification of the expression level of the GRP94 gene and/or FN14 gene comprises quantifying the messenger RNA (mRNA) of said gene, or a fragment of said mRNA, the complementary DNA (cDNA) of said gene, or a fragment of said cDNA.

**8.** The method according to claim 7, wherein the expression level is quantified by means of a quantitative polymerase chain reaction (PCR) or by means of a DNA or RNA array or by means of nucleotide hybridization techniques.

**9.** The method according to any of claims 1 to 8, wherein the sample is a tumor tissue sample.

**10.** Use of an agent suitable for the treatment of brain metastasis for manufacturing a medicinal product for the treatment or prevention of brain metastasis in a subject who has increased levels of GRP94 with respect to a reference value and has been treated with a taxane.

**11.** Use of a taxane for manufacturing a medicinal product for the treatment of brain metastasis in a subject who has decreased levels of GRP94 with respect to a reference value.

**12.** A composition comprising a taxane and a GRP94 inhibitor and/or an FN14 inhibitor, wherein if the taxane is docetaxel, then the FN14 inhibitor is not the compound of formula (I) of Table 2.

**13.** The composition according to claim 12, wherein the GRP94 inhibitor is selected from a compound of Table 1.

**14.** The composition according to any of claims 12 or 13, wherein the FN14 inhibitor is selected from a compound of Table 2.

**15.** Use of the composition according to any of claims 12 to 14 for manufacturing a medicinal product for the treatment of brain metastasis.

**16.** The method according to any of claims 1 to 9 or uses according to claims 10, 11 and 15 or composition according to any one of claims 12 to 14, wherein the taxane is selected from the group consisting of docetaxel and paclitaxel.

**17.** The method according to any of claims 4 to 9 and 16 or use according to any of claims 10 and 15, wherein the agent suitable for the treatment or prevention of brain metastasis is selected from the group consisting of an anti-angiogenic agent, hsp90 inhibitor, epidermal growth factor inhibitor, HDAC inhibitor, PARP inhibitor, BRAF inhibitor and tyrosine kinase inhibitor.

**18.** Method or use according to claim 17, wherein the anti-angiogenic agent is selected from the group consisting of bevacizumab, sunitinib, endostatin, sorafenib and cilengitide.

**19.** The method or use according to claim 17, the composition according to claim 10 or use according to claim 12, wherein, the hsp90 inhibitor is 17-(dimethylaminoethylamino)-17-demethoxygeldanamycin.

**20.** The method or use according to claim 17, wherein the epidermal growth factor inhibitor is selected from the group consisting of trastuzumab, erlotinib, lapatinib, HKI-272, afatinib, gefitinib and icotinib.

**21.** The method or use according to claim 17, wherein the HDAC inhibitor is selected from the group consisting of vorinostat and panobinostat.

**22.** The method or use according to claim 17, wherein the PARP inhibitor is selected from the group consisting of iniparib, olaparib and veliparib.

**23.** The method or use according to claim 17, wherein the BRAF inhibitor is vemurafenib.

**24.** The method or use according to claim 17, wherein the tyrosine kinase inhibitor is selected from the group consisting of sunitinib, sorafenib and pazopanib.

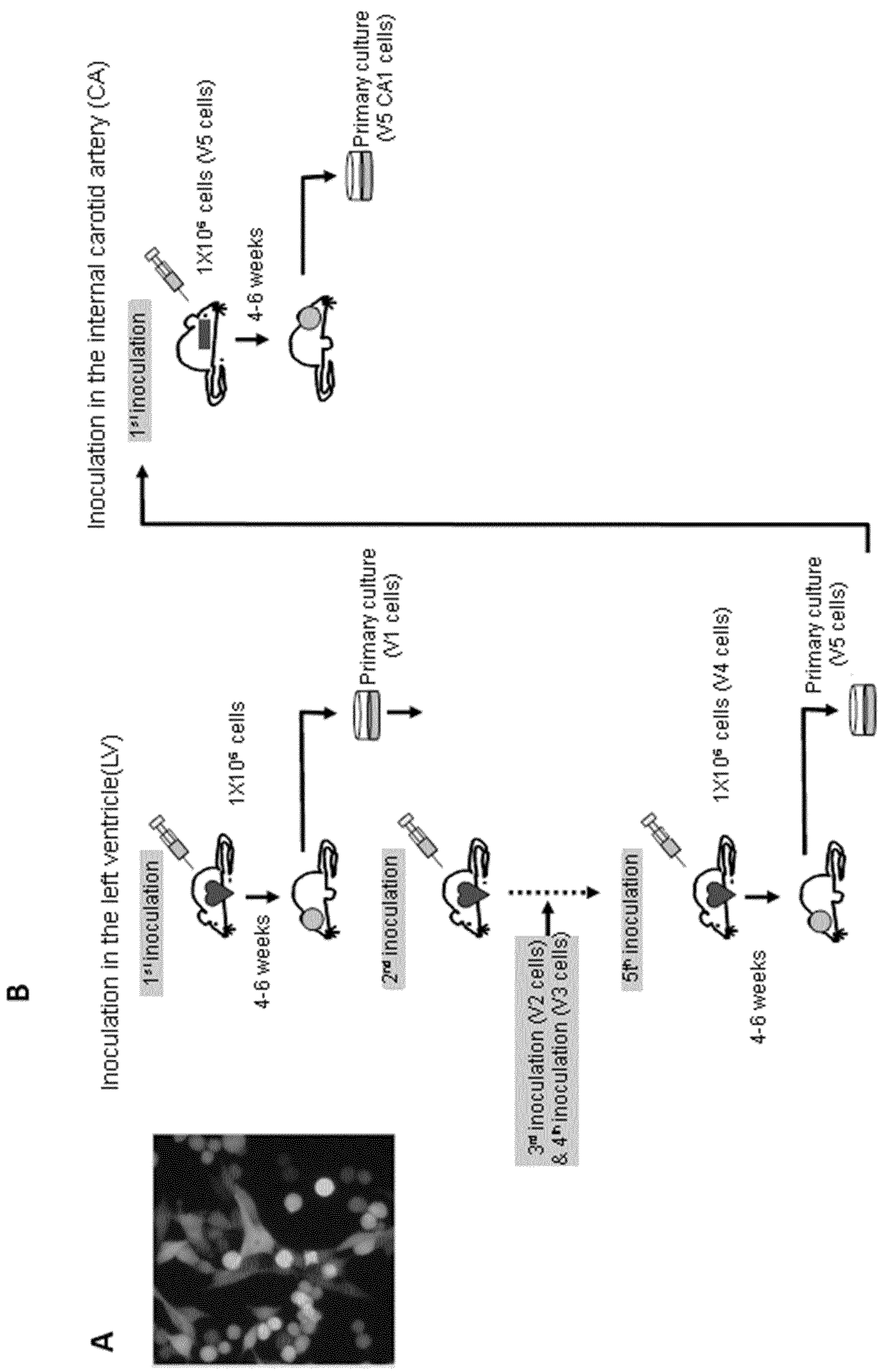
A
B
Inoculation in the left ventricle(LV)
1st inoculation
1X10^6 cells
4-6 weeks
Primary culture
(V1 cells)
2nd inoculation
3rd inoculation (V2 cells)
& 4th inoculation (V3 cells)
5th inoculation
1X10^6 cells (V4 cells)
4-6 weeks
Primary culture
(V5 cells)
Inoculation in the internal carotid artery (CA)
1st inoculation
1X10^6 cells (V5 cells)
4-6 weeks
Primary culture
(V5 CA1 cells)

Figure 1

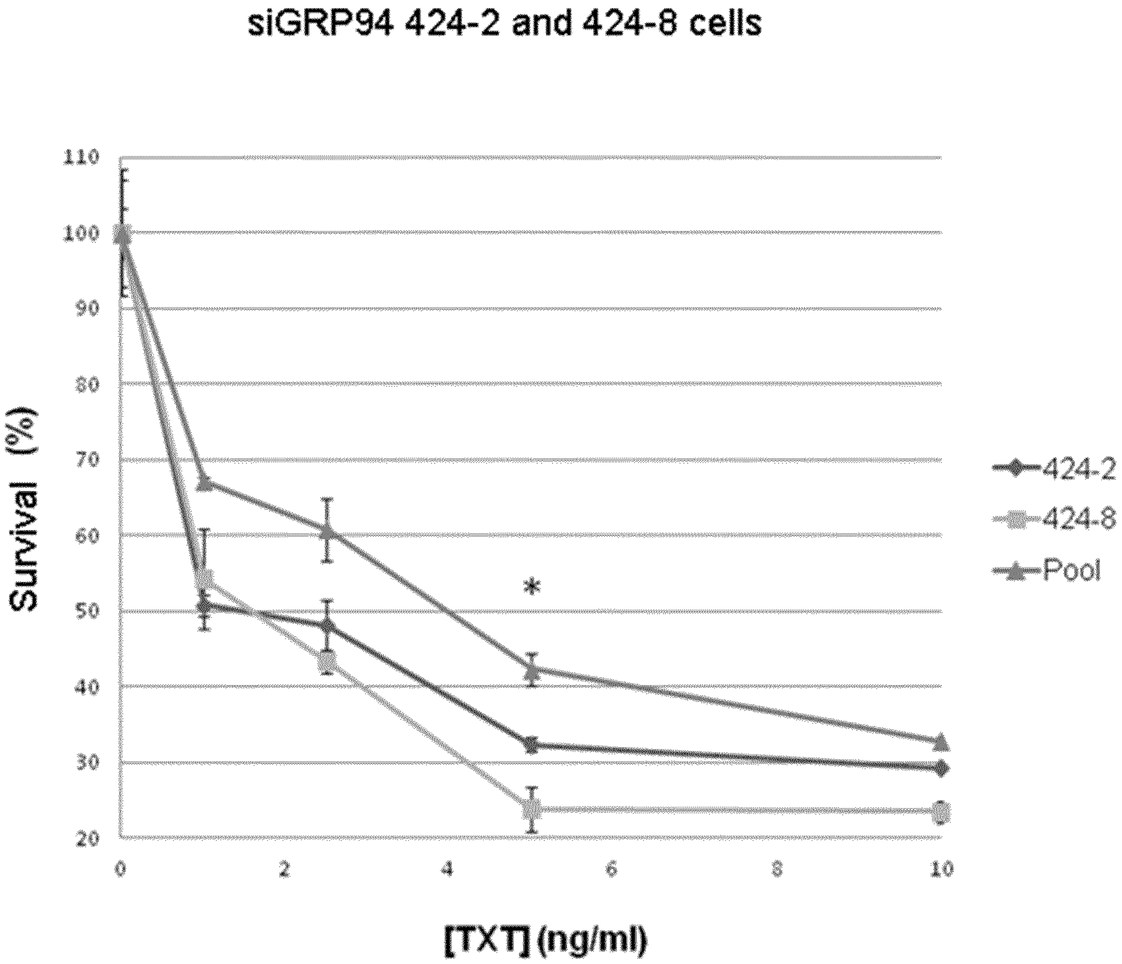


***p = 0.03 & p = 0.01**
**IC 50: 424-2** 1.5 ng/ml; **424-8** 1.59 ng/ml; **Pool** 3.95 ng/ml

Figure 2

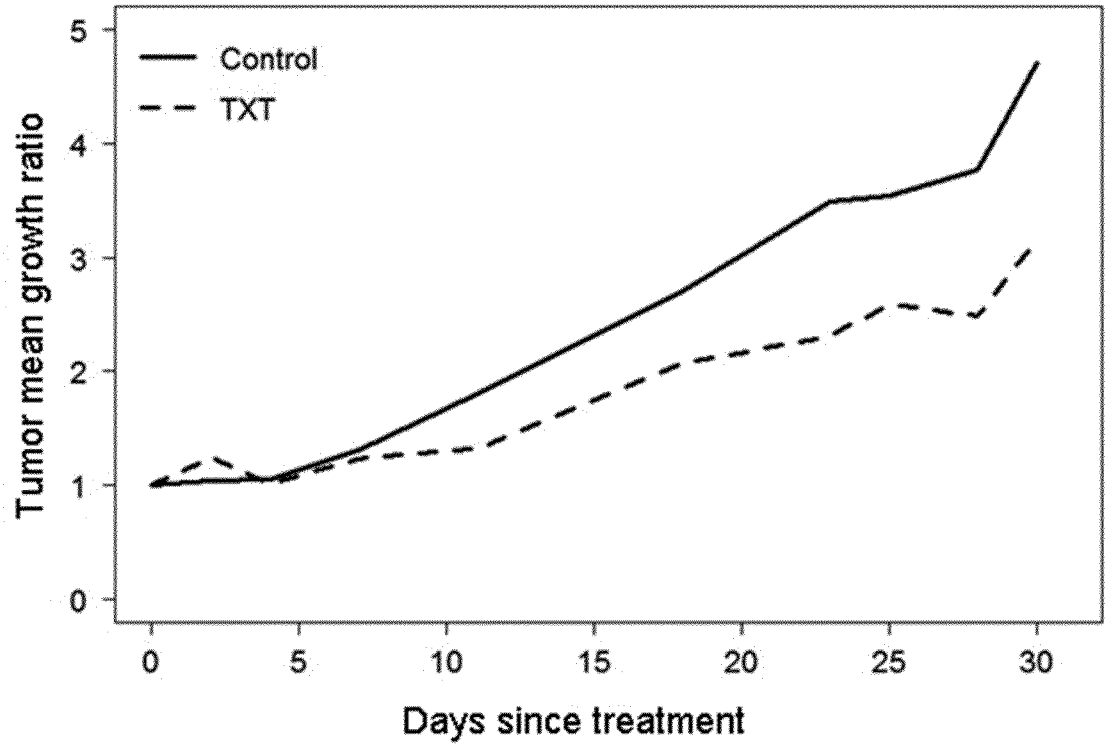
Control
TXT
Tumor mean growth ratio
0
1
2
3
4
5
0
5
10
15
20
25
30
Days since treatment

Figure 3

A

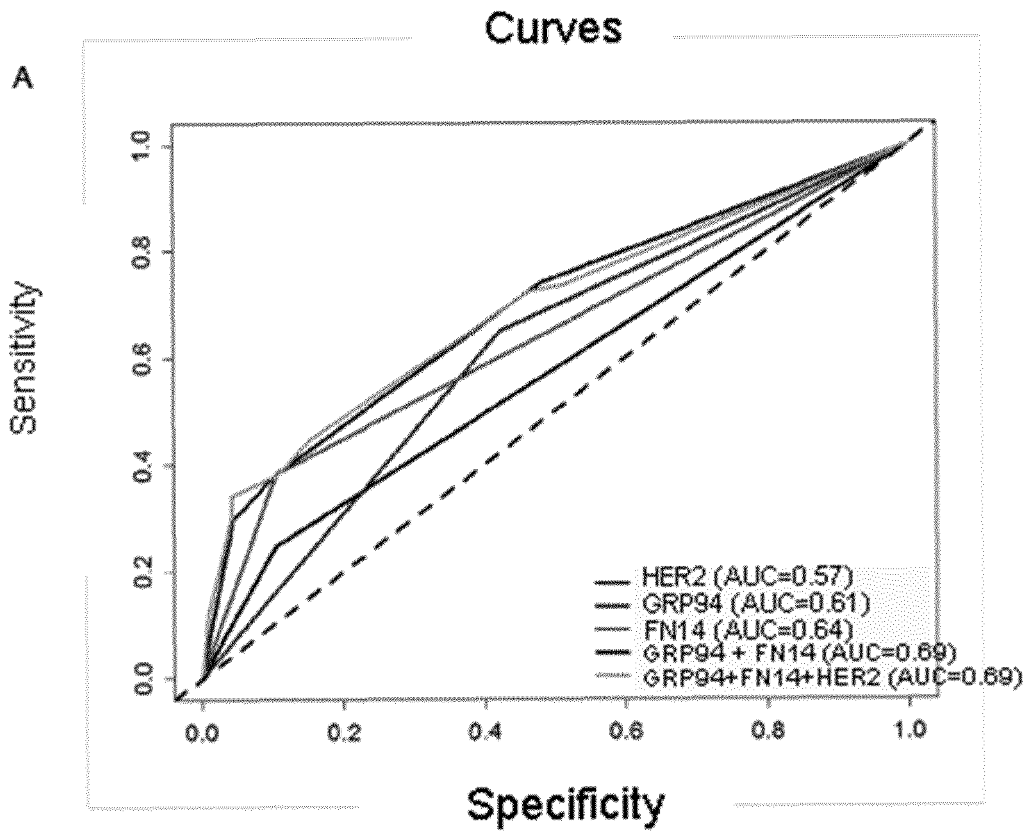
Curves
Sensitivity
1.0
0.8
0.6
0.4
0.2
0.0
HER2 (AUC=0.57)
GRP94 (AUC=0.61)
FN14 (AUC=0.64)
GRP94 + FN14 (AUC=0.69)
GRP94+FN14+HER2 (AUC=0.69)
0.0
0.2
0.4
0.6
0.8
1.0
Specificity

B

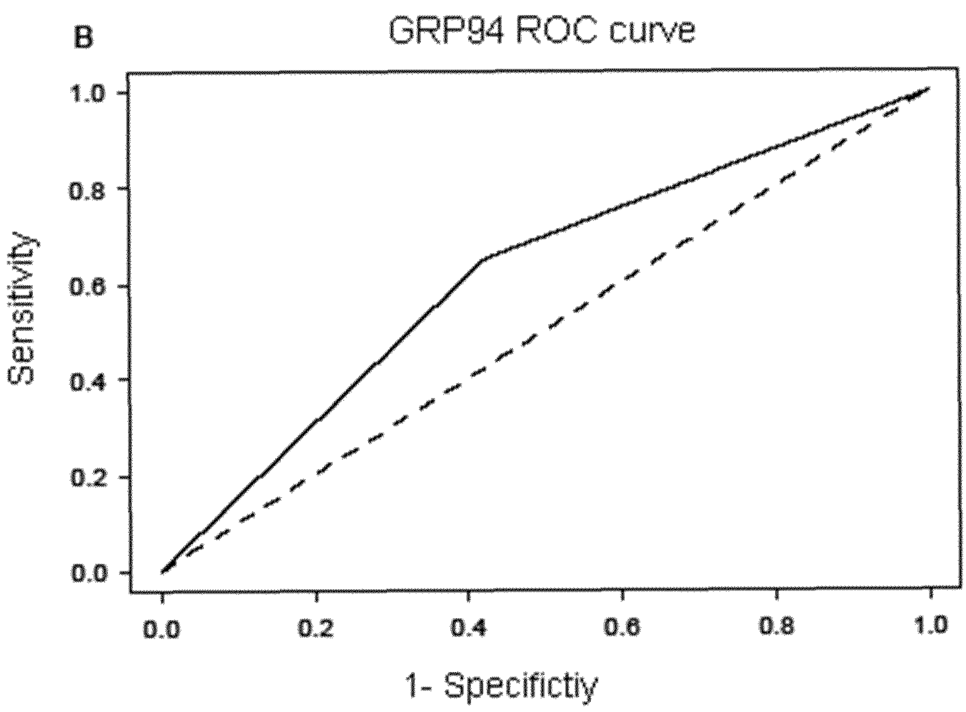
GRP94 ROC curve
1.0
0.8
0.6
0.4
0.2
0.0
Sensitivity
0.0
0.2
0.4
0.6
0.8
1.0
1- Specifictiy

Figure 4

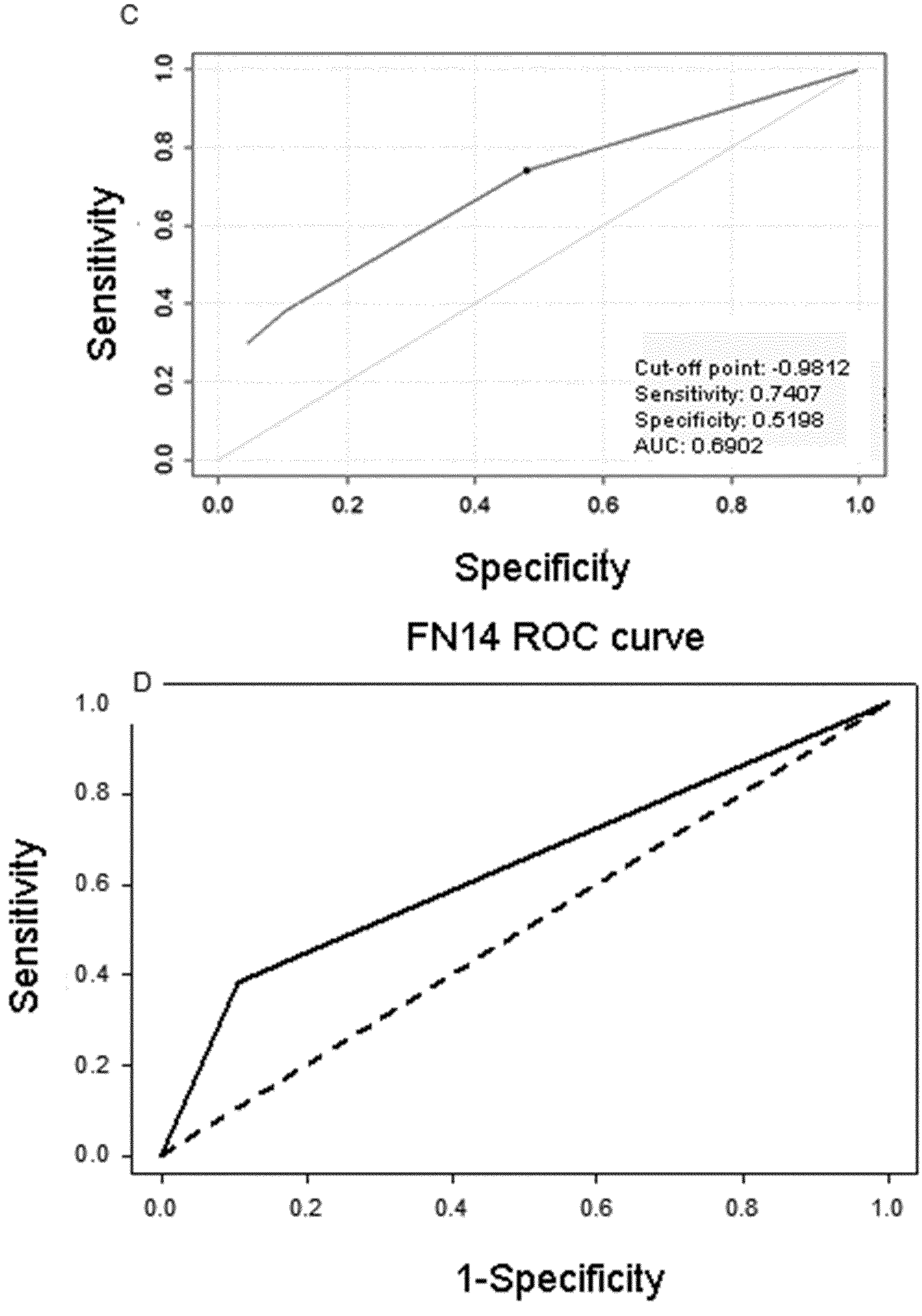
C
Sensitivity
0.0
0.2
0.4
0.6
0.8
1.0
Cut-off point: -0.9812
Sensitivity: 0.7407
Specificity: 0.5198
AUC: 0.6902
Specificity
FN14 ROC curve
D
1-Specificity

Figure 4

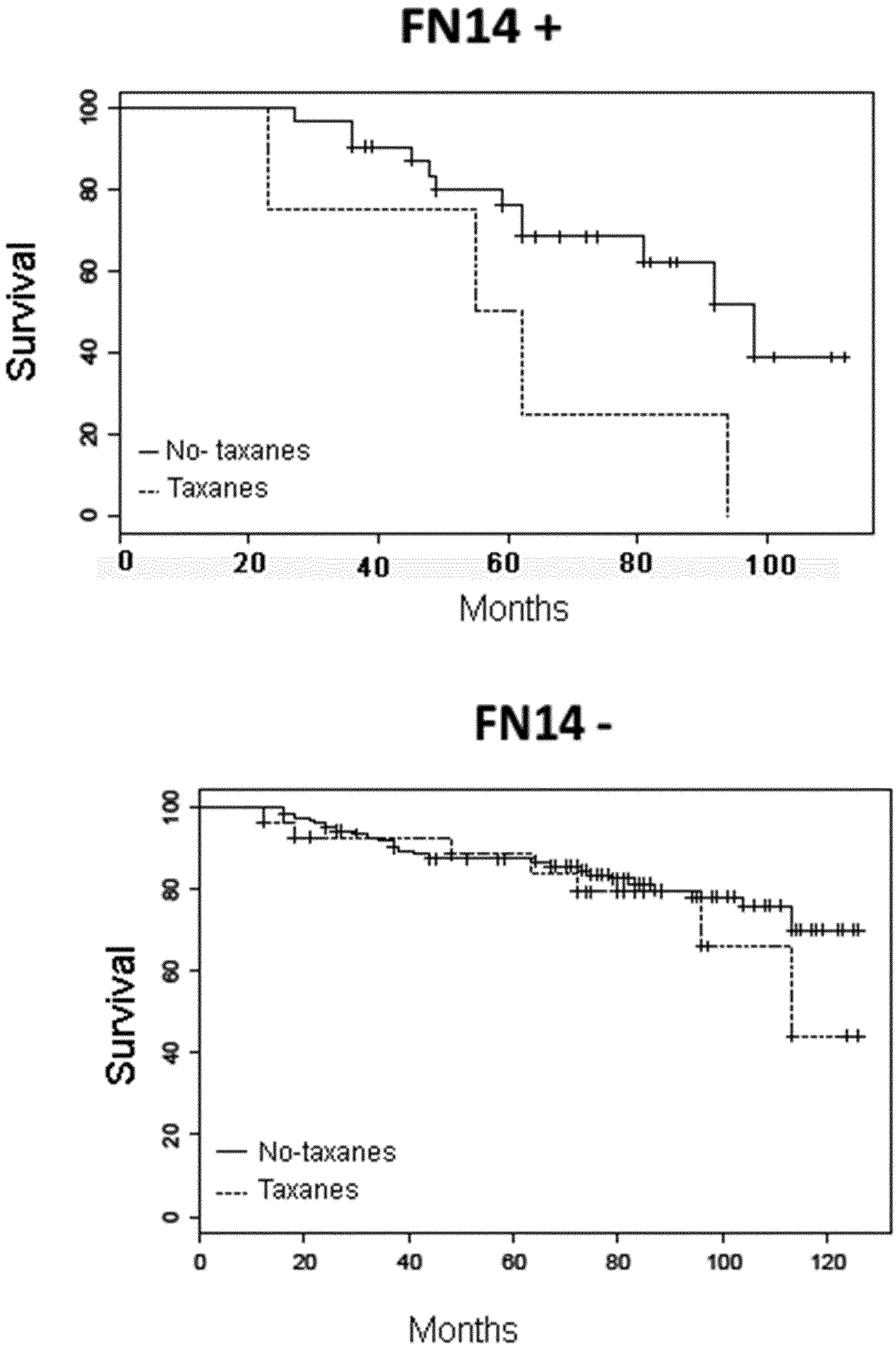
FN14 +
Survival
100
80
60
40
20
0
— No- taxanes
--- Taxanes
0
20
40
60
80
100
Months
FN14 -
Survival
100
80
60
40
20
0
— No-taxanes
---- Taxanes
0
20
40
60
80
100
120
Months

Figure 5

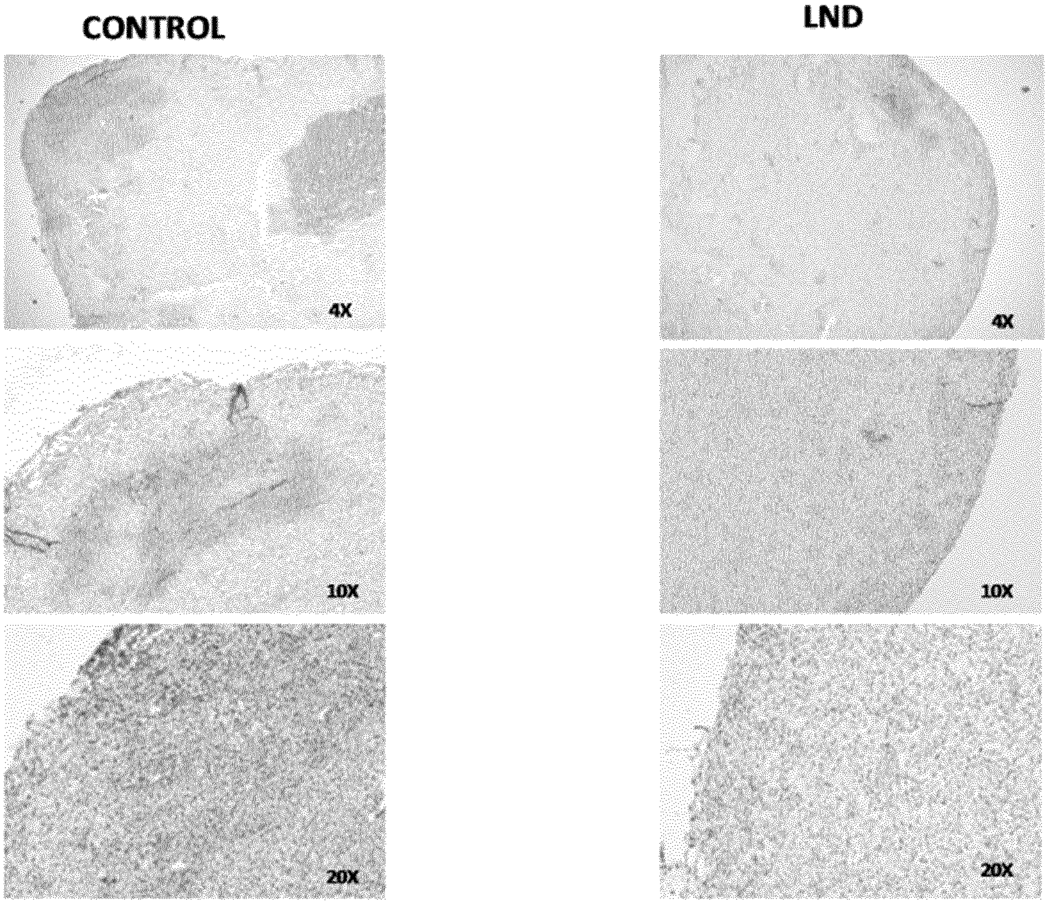


Figure 6

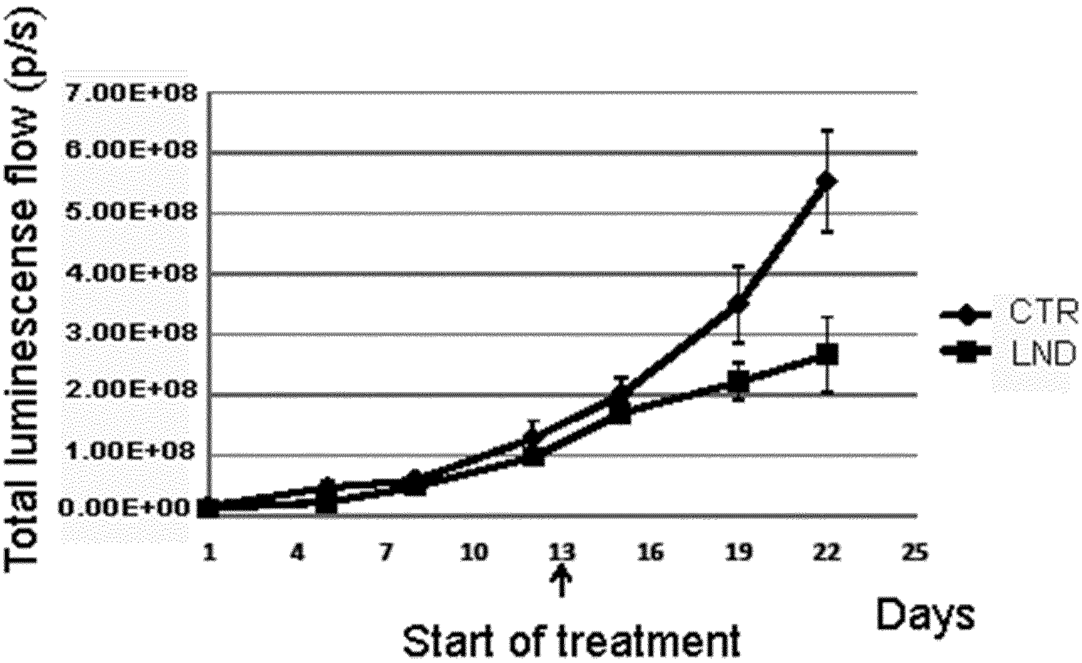
Total luminescense flow (p/s)
7.00E+08
6.00E+08
5.00E+08
4.00E+08
3.00E+08
2.00E+08
1.00E+08
0.00E+00
1
4
7
10
13
16
19
22
25
CTR
LND
Start of treatment
Days

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2330100 A1
- WO 2012052758 A1
- US 8247557 B2
- WO 2005074989 A2
- WO 2008067280 A2
- WO 2013026099 A1
- US 2012148598 A
- EP 2294089 A2
- US 20090186039 A
- US 5093246 A
- US 5176996 A
- US 5264564 A
- US 5256775 A
- WO 8809810 A
- WO 8910134 A
- US 5416016 A, Low


### Non-patent literature cited in the description


- **ZHANG L. et al.** *Science Translational Med,* 10 April 2013, vol. 5, 180ra48
- **SHAO MM. et al.** *Med Mol Morphol.,* March 2011, vol. 44 (1), 15-2
- **DOWDY ; WEARDEN.** Statistics for Research. John Wiley and Sons, 1983
- *Bioscience, Biotechnology, and Biochemistry,* 2012, vol. 76 (2), 349-352
- **DOLLINS DE et al.** *Mol Cell.,* 12 October 2007, vol. 28 (1), 41-56
- **RAMSEY, A. J. ; RUSSELL, L. C. ; WHITT, S. R. ; CHINKERS, M.** *J. Biol. Chem.,* 2000, vol. 275, 17857-17862
- **YOUNG, J. C. ; SCHNEIDER, C. ; HARTL, F. U.** *FEBS Lett.,* 1997, vol. 418, 139-143
- **BROWN SA. et al.** *Biochem J.,* 15 April 2003, vol. 371, 395-403
- **ALTSCHUL, S. et al.** *J. Mol. Biol.,* 1990, vol. 215, 403-410
- **SAMBROOK, J. et al.** Molecular cloning: a Laboratory Manual. Cold Spring Harbor Laboratory Press, vol. 1-3
- **AUERBACHJ R. et al.** *Clin Chem.,* January 2003, vol. 49 (1), 32-40
- **AHERNE W. et al.** *Methods in Molecular Medicine,* 26 March 2003, vol. 85, 149-161
- **HAWES BE et al.** *Curr Protoc Pharmacol.,* May 2001
- **LAWSON B et al.** *J Cell Physiol,* February 1998, vol. 174 (2), 170-8
- **DUERFELDT AS et al.** *J Am Chem Soc.,* 13 June 2012, vol. 134 (23), 9796-804
- **MENEZES DL et al.** *Mol Cancer Ther.,* March 2012, vol. 11 (3), 730-9
- **SEQUIST LECIA V et al.** *JCO,* 20 November 2010
- **ECCLES SA.** *Cancer Res.,* 15 April 2008, vol. 68 (8), 2850-60
- **NAKASHIMA T et al.** *Clin Cancer Res.,* 15 May 2010, vol. 16 (10), 2792-802
- **WANG Y. et al.** *Curr Opin Investig ; ;Drugs,* December 2010, vol. 11 (12), 1466-76
- **GALLERNE C. et al.** *Biochim Biophys Acta.,* June 2013, vol. 1833 (6
- **LITTLE E. et al.** *J Biol Chem.,* 21 April 1995, vol. 270 (16), 9526-34
- **DEY A. et al.** *Arch Biochem Biophys.,* 15 March 2006, vol. 447 (2), 155-66
- **MANNHOLD R. et al.** *Drug Discovery Today,* March 2010, vol. 15, 210-219
- **YUMIN XIA et al.** *Clinical Immunology,* November 2012, vol. 145, 108-121
- **MUÑOZ-GARCÍA B. et al.** *Stroke,* 2006, vol. 37, 2044-2053
- **KOHLER ; MILSTEIN et al.** *Nature,* 1975, vol. 256, 495
- **ROSSI.** *Current Biology,* 1994, vol. 4, 469-471
- **ZAUG et al.** *Science,* 1984, vol. 224, 574-578
- **VAN DER KROL et al.** *BioTechniques,* 1988, vol. 6, 958-976
- **STEIN et al.** *Cancer Res,* 1988, vol. 48, 2659-2668
- **WAGNER.** *Nature,* 1994, vol. 372, 333
- **LETSINGER et al.** *Proc. Natl. Acad. Sci. U.S.A.,* 1989, vol. 86, 6553-6556
- **LEMAITRE et al.** *Proc. Natl. Acad. Sci.,* 1987, vol. 84, 648-652
- **ZON.** *Pharm. Res.,* 1988, vol. 5, 539-549
- **PERRY-O'KEEFE et al.** *Proc. Natl. Acad. Sci. U.S.A.,* 1996, vol. 93, 14670
- **EGLOM et al.** *Nature,* 1993, vol. 365, 566
- **HELENE.** *Anticancer Drug Des.,* 1991, vol. 6 (6), 569-84

- **GYNTHER M.** Publications of the University of Eastern Finland, Dissertations. Health Sciences, 2010
- *Lundquist and Renftel Vascul Pharmacol,* 2002, vol. 38, 355-364
- **TAKASATO et al.** *Am J Physiol,* 1984, vol. 247, H484-493
- **DE LANGE et al.** *Adv Drug Deliv Rev,* 1999, vol. 36, 211-227
- **RAUTIO et al.** *Aaps J,* 2008, vol. 10, 92-102
- Microcapsules and nanoparticles in Medicine and Pharmacy. CRC Press, 1992
- **MATHIOWITZ ; LANGER.** *J. Control. Release,* 1987, vol. 5, 13
- **MATHIOWITZ et al.** *Reactive Polymers,* 1987, vol. 6, 275
- **MATHIOWITZ et al.** *J. Appl. Polymer Sci.,* 1988, vol. 35, 755
- **LANGER.** *Ace. Chem. Res.,* 2000, vol. 33, 94
- **LANGER.** *J. Control. Release,* 1999, vol. 62, 7
- **UHRICH et al.** *Chem. Rev.,* 1999, vol. 99, 3181
- **ZHOU et al.** *J. Control. Release,* 2001, vol. 75, 27
- **HANES et al.** *Pharm. Biotechnol.,* 1995, vol. 6, 389
- **V.V. RANADE.** *J. Clin. Pharmacol.,* 1989, vol. 29, 685
- **UMEZAWA et al.** *Biochem. Biophys. Res. Comman.,* 1988, vol. 153, 1038
- **P.G. BLOEMAN et al.** *FEBS Lett.,* 1995, vol. 357, 140
- **M. OWAIS et al.** *Antimicrob. Agents Chemother,* 1995, vol. 39, 180
- **BRISCOE et al.** *Am. J. Physiol.,* 1995, vol. 1233, 134
- **SCHREIER et al.** *J. Biol. Chem.,* 1994, vol. 269, 9090
- **K. KEINANEN ; MX. LAUKKANEN.** *FEBS Lett.,* 1994, vol. 346, 123
- **J.J. KILLION ; I.J. FIDLER.** *Immunomethods,* 1994, vol. 4, 273
- **MARTINEZ-ARANDA A.** *Int. J. Mol. Sci.,* 2013, vol. 14 (4), 8306-8327
- **BIN GU et al.** *Carcinogenesis,* 2004
- **SANZ et al.** *Am J Pathol,* 2011, vol. 179 (2), 564-579